(19) Europäisches Patentamt / European Patent Office / Office européen des brevets

(11) **EP 1 944 296 A1**

(12) **EUROPEAN PATENT APPLICATION**


(43) Date of publication:
**16.07.2008 Bulletin 2008/29**

(21) Application number: **07384006.8**

(22) Date of filing: **15.01.2007**

(51) Int Cl.:
***C07D 231/06*** *(2006.01)* ***A61K 31/4155*** *(2006.01)*
***A61P 25/00*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**



(71) Applicant: **Laboratorios del Dr. Esteve S.A.**
**08041 Barcelona (ES)**

(72) Inventors:
- **Buschmann, Helmut H. Dr.**
  **08960 Sant Just Desvem (ES)**
- **Torrens-Jover, Antonio**
  **08221 Terrassa (Barcelona) (ES)**
- **Yenes-Minguez, Susana**
  **08750 Molins de Rei (Barcelona) (ES)**
- **Benet-Bucholz, Jordi**
  **43893 Altafulla (Taragona) (ES)**
- **Sola-Carandell, Lluis**
  **43893 Altafulla (Taragona) (ES)**

(74) Representative: **Peters, Hajo**
**Bosch Graf von Stosch Jehle**
**Patentanwaltsgesellschaft mbH**
**Flüggenstrasse 13**
**80639 München (DE)**


(54) **Amorphous phase of a substituted pyrazoline, its preparation and use as medicaments**


(57) The present invention relates to the amorphous phase of (R)-N-piperidinyl-5(4- chlorophenyl)-1-(2,4-dichlorophenyl)-4,5-dihydro-1H-pyrazole-3-carboxamide,

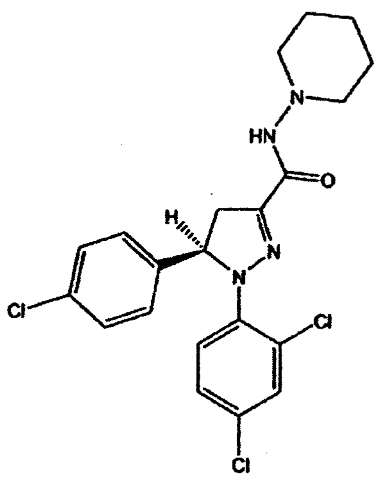

methods for its preparation, medicaments comprising this compound as well as their use for the preparation of a medicament for the treatment of humans and animals. It has been found that these compounds have a high affinity for cannabinoid receptors, particularly for the $CB_1$-receptor, and that they act as modulators e.g. antagonists, inverse agonists or agonists on these receptors. They are therefore suitable for the prophylaxis and/or treatment of various disorders related to the central nervous system, the immune system, the cardiovascular system, the endocrinous system, the respiratory system, the gastrointestinal tract or reproduction in humans and/or animals, preferably humans including infants, children and grown-ups.


EP 1 944 296 A1

## Description

The present invention relates to an amorphous phase of a substituted pyrazoline, methods for its preparation, medicaments comprising the compound as well as use for the preparation of a medicament for the treatment of humans and animals.

Cannabinoids are compounds, which are derived from the cannabis sativa plant which is commonly known as marijuana. The most active chemical compound of the naturally occurring cannabinoids is tetrahydrocannabinol (THC), particularly $\Delta^9$-THC.

These naturally occuring cannabinoids as well as their synthetic analogues promote their physiological effects via binding to specific G-coupled receptors, the so-called cannabinoid-receptors.

At present, two distinct types of receptors that bind both the naturally occurring and synthetic cannabinoids have been identified and cloned. These receptors, which are designated $CB_1$ and $CB_2$ are involved in a variety of physiological or pathophysiological processes in humans and animals, e.g. processes related to the central nervous system, immune system, cardiovascular system, endocrinous system, respiratory system, the gastrointestinal tract or to reproduction, as described for example, in Hollister, Pharm. Rev. 38, 1986, 1-20; Reny and Singha, Prog. Drug. Res., 36, 71-114, 1991; Consroe and Sandyk, in Marijuana/Cannabinoids, Neurobiology and Neurophysiology, 459, Murphy L. and Barthe A. Eds., CRC Press, 1992.

Therefore, compounds, which have a high binding affinity for these cannabinoid receptors and which are suitable for modulating these receptors are useful in the prevention and/or treatment of cannabinoid-receptor related disorders.

In particular, the $CB_1$-Receptor is involved in many different food-intake related disorders such as bulimia or obesity, including obesity associated with type II diabetes (non-insulin-dependent diabetes) and thus, compounds suitable for regulating this receptor may be used in the prophylaxis and/or treatment of these disorders.

Thus, it was an object of the present invention to provide novel compounds for use as active substances in medicaments. In particular, these active substances should be suitable for the modulation of Cannabinoid receptors, more particularly for the modulation of Cannabinoid 1 ($CB_1$) receptors.

Said object was achieved by providing the amorphous phase of the piperidinyl-pyrazoline compound given below.

It has been found that these compounds have a high affinity for cannabinoid receptors, particularly for the $CB_1$-receptor, and that they act as modulators e.g. antagonists, inverse agonists or agonists on these receptors. They are therefore suitable for the prophylaxis and/or treatment of various disorders related to the central nervous system, the immune system, the cardiovascular system, the endocrinous system, the respiratory system, the gastrointestinal tract or reproduction in humans and/or animals, preferably humans including infants, children and grown-ups.

Thus, in one of its aspects the present invention relates to the amorphous phase of (R)-N-piperidinyl-5-(4-chlorophenyl)-1-(2,4-dichlorophenyl)-4,5-dihydro-1H-pyrazole-3-carboxamide.

This amorphous phase of (R)-N-piperidinyl-5-(4-chlorophenyl)-1-(2,4-dichlorophenyl)-4,5-dihydro-1H-pyrazole-3-carboxamide shows a good stability under certain conditions and/or over time, eg. in watery solutions and/or at room temperature. It also shows a relatively good solubility.

As preferred embodiment the of the present invention the amorphous phase of (R)-N-piperidinyl-5-(4-chlorophenyl)-1-(2,4-dichlorophenyl)-4,5-dihydro-1H-pyrazole-3-carboxamide shows a glass transition in the DSC analysis at 64.5 $\pm$ 4 °C, preferably at 64.5 $\pm$ 3 °C, more preferably at 64.5 $\pm$ 2 °C or also between 63 and 66° C.

As another preferred embodiment of the present invention the amorphous phase of (R)-N-piperidinyl-5-(4-chlorophenyl)-1-(2,4-dichlorophenyl)-4,5-dihydro-1H-pyrazole-3-carboxamide decomposes at or above 300 °C.

As another preferred embodiment of the present invention the amorphous phase of (R)-N-piperidinyl-5-(4-chlorophenyl)-1-(2,4-dichlorophenyl)-4,5-dihydro-1H-pyrazole-3-carboxamide shows in the FTIR spectrum peaks, preferably intense and/or broad peaks at 1656 $\pm$ 5 $cm^{-1}$ and/or 1473 $\pm$ 5 $cm^{-1}$ and no peaks, preferably no intense peaks, at 3325 $\pm$ 10 $cm^{-1}$.

As another preferred embodiment of the present invention the amorphous phase of (R)-N-piperidinyl-5-(4-chlorophenyl)-1-(2,4-dichlorophenyl)-4,5-dihydro-1H-pyrazole-3-carboxamide shows in the FTRaman spectrum bands, preferably intense and/or broad bands, at 1666 $\pm$ 5 $cm^{-1}$.

It is highly preferred, if the amorphous phase of (R)-N-piperidinyl-5-(4-chlorophenyl)-1-(2,4-dichlorophenyl)-4,5-dihydro-1H-pyrazole-3-carboxamide at least 2, preferably at leat 3, more preferably all 4 attributes from the following list:

- it shows a glass transition in the DSC analysis at 64.5 $\pm$ 4°C, preferably at 64.5 $\pm$ 3 °C, more preferably at 64.5 $\pm$ 2 °C or also between 63 and 66° C;
- it decomposes at or above 300 °C;

- it shows in the FTIR spectrum peaks, preferably intense and/or broad peaks at 1656 ± 5 $cm^{-1}$ and/or 1473 ± 5 $cm^{-1}$ and no peaks, preferably no intense peaks, at 3325 ± 10 $cm^{-1}$;

- it shows in the FTRaman spectrum bands, preferably intense and/or broad bands, at 1666 ± 5 $cm^{-1}$.

In another aspect the present invention also provides a process for the preparation of the amorphous phase of (R)-N-piperidinyl-5-(4-chlorophenyl)-1-(2,4-dichlorophenyl)-4,5-dihydro-1 H-pyrazole-3-carboxamide from (R)-N-piperidinyl-5-(4-chlorophenyl)-1-(2,4-dichlorophenyl)-4,5-dihydro-1H-pyrazole-3-carboxamide, wherein (R)-N-piperidinyl-5-(4-chlorophenyl)-1-(2,4dichlorophenyl)-4,5-dihydro-1H-pyrazole-3-carboxamide is melted, preferably at a temperature between 78 and 88°C, and subsequently cooled below the melting point, preferably to room temperature.

(R)-N-piperidinyl-5-(4-chlorophenyl)-1-(2,4-dichlorophenyl)-4,5-dihydro-1H-pyrazole-3-carboxamide may be prepared by reacting a compound of formula IIa

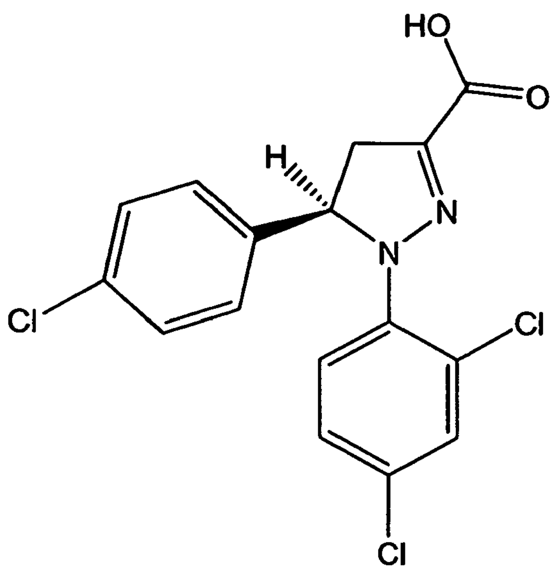

IIa

is optionally transferred under inert atmosphere to a compound of general formula (IIIa) via reaction with an activating agent

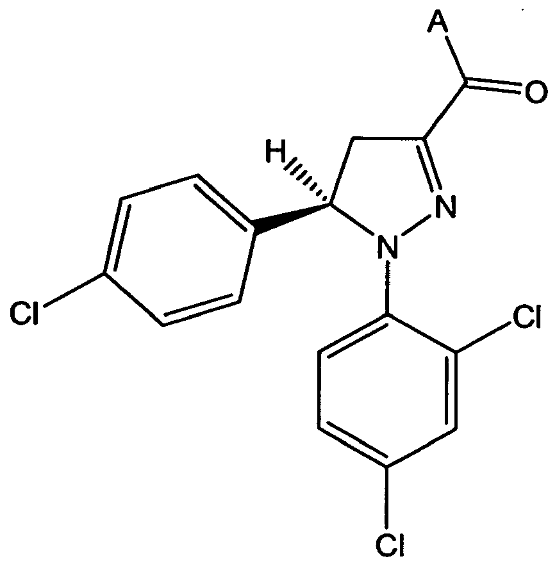

, IIIa

wherein A represents a leaving group, preferably a chlorine atom, said compound being optionally isolated and/or optionally purified, and one compound of formula (IIa) or general formula (IIIa) is reacted with a compound of formula VIII,

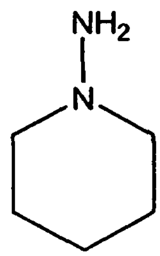

VIII

under inert atmosphere to yield (R)-N-piperidinyl-5-(4-chlorophenyl)-1-(2,4-dichlorophenyl)-4,5-dihydro-1H-pyrazole-3-carboxamide of formula (I),

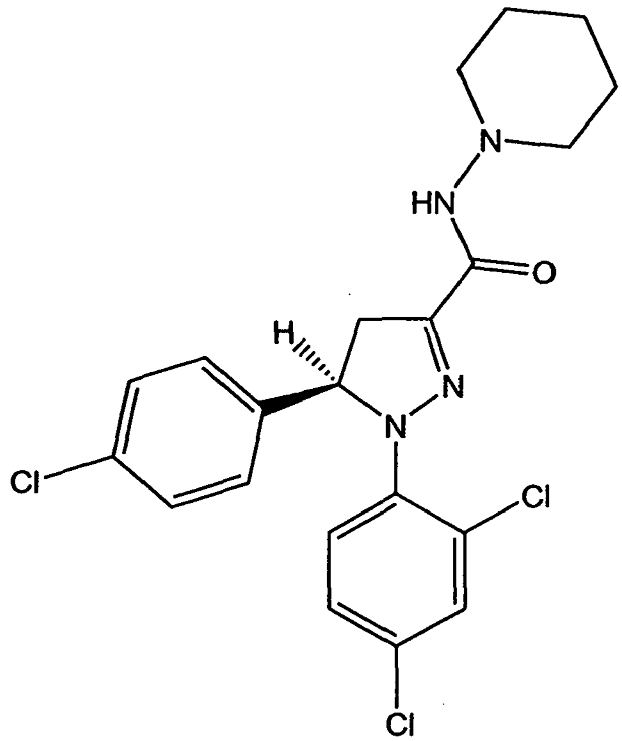

which is optionally isolated and/or optionally purified.

Preferably the compound IIa is obtained from a mixture comprising the enantiomers

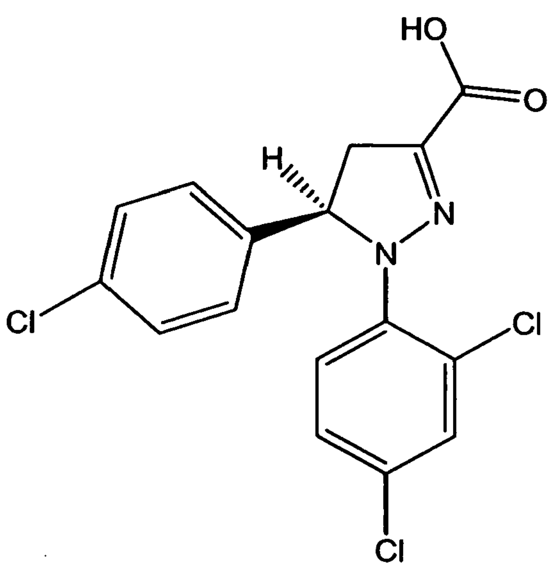

IIa

and

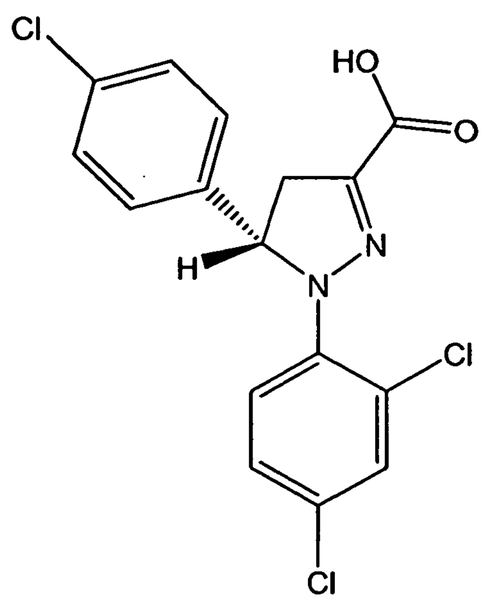

IIb

The compound (IIa) may be obtained from the mixture of the enantiomers by means well known to those skilled in the art. Preferably, the compound IIa is obtained from the mixture in form of an addition compound with a chiral base,

preferably (+)-Cinchonine or R-(+)-1-Phenylethylamine, and preferably liberated from the addition compound.

Preferably the racemic mixture according to formula II

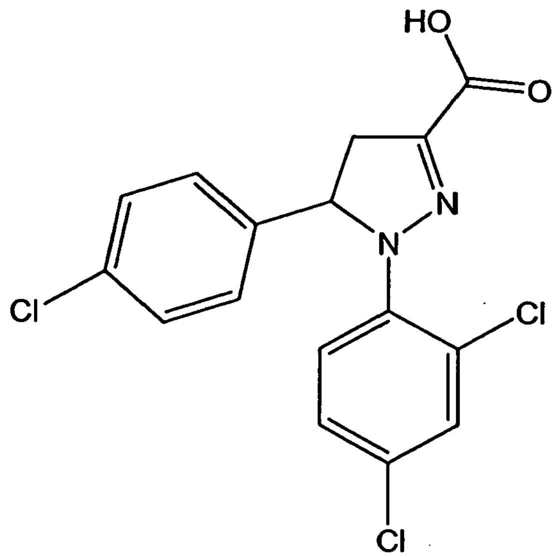

II

comprising the enantiomers IIa and IIb may be obtained by reacting one benzaldehyde compound of general formula IV

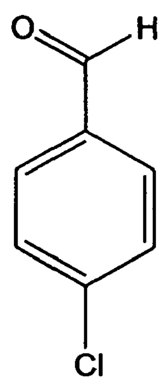

(IV)

with a pyruvate compound of formula (V)

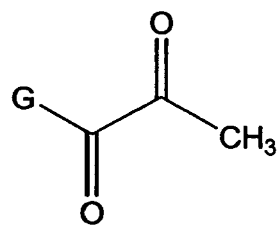

(V),

wherein G represents an OR group with R being a branched or unbranched $C_{1-6}$ alkyl radical or G represents an $O^-K$ group with K being a cation,
to yield a compound of formula (VI)

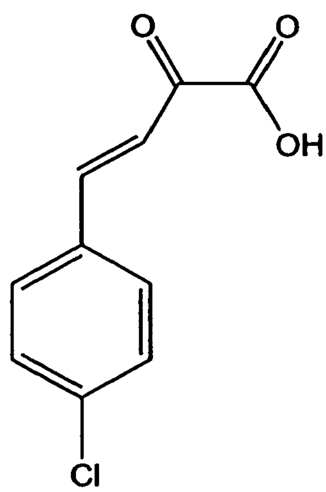

(VI)

which is optionally isolated and/or optionally purified, and which is reacted with an optionally substituted phenyl hydrazine of formula (VII)

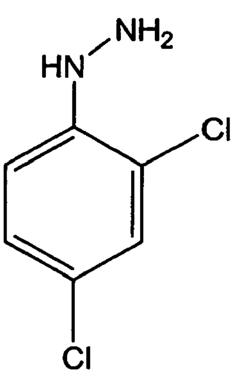

(VII)

or a corresponding salt thereof, under inert atmosphere, to yield a compound of formula (II)

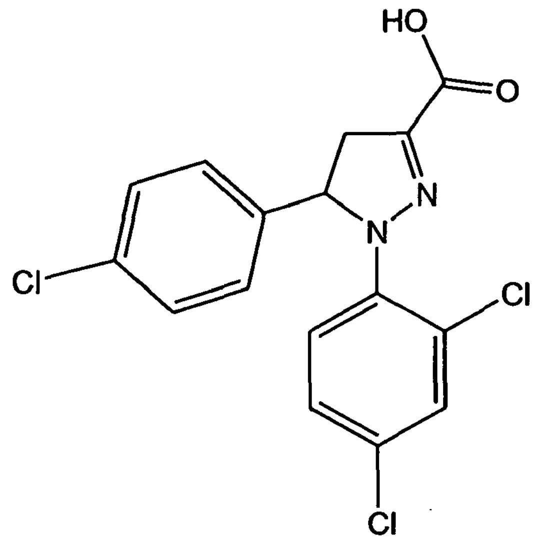

(II)

which is optionally isolated and/or optionally purified.

The production of the compound according to formula II is already described in detail in international patent applications WO2005/077909 and WO 2005/077911. The respective descriptions are hereby incorporated by reference and form part of the present disclosure.

The reaction of general formula (IIa) with a compound of formula VIII to yield compounds of general formula I, is preferably carried out in presence of a base such as triethylamine in a reaction medium such as methylenchloride. The temperature is preferably in the range from 0˚C to the boiling point of the reaction medium. The reaction time may vary over a broad range, e.g. from several hours to several days.

The aforementioned reaction involving the synthesis of the 4,5-dihydro-pyrazole ring preferably is carried out under an inert atmosphere, preferably nitrogen or argon, to avoid oxidation of the ring-system.

During the processes described above the protection of sensitive groups or of reagents may be necessary and/or desirable. The introduction of conventional protective groups as well as their removal may be performed by methods well-known to those skilled in the art.

The purification and isolation of (R)-N-piperidinyl-5-(4-chlorophenyl)-1-(2,4-dichlorophenyl)-4,5-dihydro-1H-pyrazole-3-carboxamide or any intermediate thereof may, if required, be carried out by conventional methods known to those skilled in the art, e.g. chromatographic methods or recrystallization.

The amorphous phase according to the invention, is suitable as pharmaceutical active substance for the preparation of medicaments.

It has been found that the amorphous phase has a high affinity to cannabinoid receptors, particularly cannabinoid 1 ($CB_1$)-receptors, i.e. it is a selective ligand for the ($CB_1$)-receptor and act as modulators, e.g. antagonists, inverse agonists or agonists, on these receptors. In particular, the compound shows little or no development of tolerance during treatment, particularly with respect to food intake, i.e. if the treatment is interrupted for a given period of time and then continued afterwards, the inventively used compound will again show the desired effect. After ending the treatment with the compound, the positive influence on the body weight is found to continue at least for a certain period of time.

Furthermore, this amorphous phase is connected to relatively weak Herg channel affinity, thus a low risk of prolongation of the QT-interval is to be expected for this compound.

In summary, the inventively used polymorp is distinguished by a broad spectrum of beneficial effects, while at the same time showing relatively little undesired effects, i.e. effects which do not positively contribute to or even interfere with the well being of the patient.

Thus, another aspect of the present invention relates to a medicament comprising the amorphous phase of (R)-N-piperidinyl-5-(4-chlorophenyl)-1-(2,4-dichlorophenyl)-4,5-dihydro-1 H-pyrazole-3-carboxamide according to the invention, and optionally one or more pharmaceutically acceptable excipients.

Another aspect of the present invention is the use of the amorphous phase of (R)-N-piperidinyl-5-(4-chlorophenyl)-1-(2,4-dichlorophenyl)-4,5-dihydro-1H-pyrazole-3-carboxamide according to the invention, and optionally one or more pharmaceutically acceptable excipients, for the preparation of a medicament for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

Also preferred is the use of the amorphous phase of (R)-N-piperidinyl-5-(4-chlorophenyl)-1-(2,4-dichlorophenyl)-4,5-dihydro-1 H-pyrazole-3-carboxamide according to the invention, and optionally one or more pharmaceutically acceptable excipients, for the prophylaxis and/or treatment of psychosis.

Also particularly preferred is the use of the amorphous phase of (R)-N-piperidinyl-5-(4-chlorophenyl)-1-(2,4-dichlorophenyl)-4,5-dihydro-1H-pyrazole-3-carboxamide according to the invention, and optionally one or more pharmaceutically acceptable excipients, for the preparation of a medicament for the prophylaxis and/or treatment of food intake disorders, preferably bulimia, anorexia, cachexia, obesity and/or type II diabetus mellitus (non-insuline dependent diabetes mellitus), more preferably obesity.

Also preferred is the use of the amorphous phase of (R)-N-piperidinyl-5-(4-chlorophenyl)-1-(2,4-dichlorophenyl)-4,5-dihydro-1H-pyrazole-3-carboxamide according to the invention, and optionally one or more pharmaceutically acceptable excipients, for the preparation of a medicament for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

Also preferred is the use of the amorphous phase of (R)-N-piperidinyl-5-(4-chlorophenyl)-1-(2,4-dichlorophenyl)-4,5-dihydro-1 H-pyrazole-3-carboxamide according to the invention, and optionally one or more pharmaceutically acceptable excipients, for the preparation of a medicament for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

Medicaments/drugs, which are frequently the subject of misuse include opioids, barbiturates, cannabis, cocaine, amphetamines, phencyclidine, hallucinogens and benzodiazepines.

Also preferred is the use of the amorphous phase of (R)-N-piperidinyl-5-(4-chlorophenyl)-1-(2,4-dichlorophenyl)-4,5-dihydro-1H-pyrazole-3-carboxamide according to the invention, and optionally one or more pharmaceutically acceptable excipients, for the preparation of a medicament for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, , medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

Also particularly preferred is the use of the amorphous phase of (R)-N-piperidinyl-5-(4-chlorophenyl)-1-(2,4-dichlorophenyl)-4,5-dihydro-1H-pyrazole-3-carboxamide according to the invention, optionally in form of one of the stereoisomers, preferably enantiomers or diastereomers, a racemate or in form of a mixture of at least two of the stereoisomers, preferably enantiomers and/or diastereomers, in any mixing ratio, or a corresponding solvate thereof, and optionally one or more pharmaceutically acceptable excipients, for the preparation of a medicament for the prophylaxis and/or treatment of one or more disorders selected from the group consisting of dementia and related disorders, preferably for the prophylaxis and/or treatment of one or more types of dementia selected from the group consisting of memory loss, vascular dementia, mild cognitive impairment, frontotemporal dementia and Pick's disease; binge eating disorders; juvenile obesity; drug induced obesity; atypical depression; behavioural addictions; attention deficit disorders; Tourette's syndrome; suppression of reward-related behaviours; e. g. conditioned place avoidance such as suppression of cocaine- and morphine induced conditioned place preference; impulsivity; sexual dysfunction; preferably for the prophylaxis and/or treatment of one or more types of sexual dysfunction selected from the group consisting of erectile difficulty and female sexual dysfunction; seizure disorders; nausea; emesis; neuroinflammatory disease, preferably for the prophylaxis and/or treatment of one or more types of neuroinflammatory diseases selected from the group consisting of multiple sclerosis, demyelinisation related disorders, Guillan-Barré syndrome, viral encephalitis and cerebrovascular accidents; neurological disorders; muscle spasticity; traumatic brain injury; spinal cord injury; inflammation and immunomodulatory disorders, preferably for the treatment and/or prophylaxis of one or more types of inflammation and immunomodulatory disorders selected from the group consisting of cutaneous T-cell lymphoma, rheumatoid arthritis, systemic lupus erythematosus, sepsis, sarcoidosis, idiopathic pulmonary fibrosis, bronchopulmonary dysplasia, retinal

disease, scleroderma, renal ischemia, mycocardial infarction, cerebral ischemia, nephritis, hepatitis, glomerulonephritis, cryptogenic fibrosing aveolitis, psoriasis, transplant rejection, atopic dermatitis, vasculitis, allergy, seasonal allergic rhinitis, Crohn's disease, inflammatory bowel disease, reversible airway obstruction, adult respiratory distress syndrome, chronic obstructive pulmonary disease and bronchitis; cerebral apoplexy; craniocerebral trauma; neuropathic pain disorders; gastric ulcers; atheriosclerosis and liver cirrhosis.

Dementia is a disease characterized by the progressive deterioration in cognitive and social adaptive functions that can eventually interfere with the patient's ability to live independently. Dementia also constitutes of impairment in short- and long-term memory plus additional symptoms, such as problems with abstract thinking, judgment, or personality. An estimated 18 million patients suffer from dementia worldwide. The most common forms of dementia include Alzheimer's disease and vascular dementia. Other forms are frontotemporal dementia and Pick's disease.

Dementia can also be of vascular origin. Vascular dementia (atherosclerotic cerebrovascular disease) is considered to be the second most common dementia of late life, affecting approximately 10-15% of all cases. Alzheimer's disease (AD) and vascular dementia can exist in isolation or together (mixed dementia). In vascular dementia, atherosclerotic changes in cerebral vessels can lead to reduced local blood flow that results in multiple small strokes (multi-infarct dementia). Vascular dementia is pharmacologically treated by stroke prophylaxis, and by treatment of the cognitive deficit.

Alzheimer's disease (AD), the most common and important form of dementia, is a neurodegenerative disorder that is characterized by progressive impairment of cognitive functions, such as abstract reasoning and memory. Currently, an estimated 2 million people in the United States and 12 million worldwide are afflicted by this disease. Due to increasing life expectancy, it is predicted that there will be over 100 million AD patients worldwide by the year 2050. AD is one of the most prevalent illnesses in the elderly. The majority of AD patients are in their sixties or older. More than 5% of all persons over the age of 70 have significant memory loss due to AD.

AD is mainly characterized through a gradual development of forgetfulness. In further advanced disease stages, other failures in cerebral function become increasingly apparent. This includes impairment of speech, writing, and arithmetic skills. Visiospacial orientation, such as parking the car, dressing properly, and giving and understanding directions to a location, can become defective or impaired. In late stage disease, patients forget how to use common objects and tools while retaining necessary motor power and co-ordination for these activities.

Schizophrenia is characterized by profound disruption in cognition and emotion, affecting the most fundamental human attributes: language, thought, perception, affect, and sense of self. Positive symptoms include psychotic manifestations, such as hearing internal voices or experiencing other sensations not connected to an obvious source (hallucinations) and assigning unusual significance or meaning to normal events or holding fixed false personal beliefs (delusions). Negative symptoms are characterized by affective flattening and lack of initiative or goals (avolition), loss of usual interests or pleasures (anhedonia), disturbances of sleep and eating, dysphoric mood (depressed, anxious, irritable, or angry mood) and difficulty concentrating or focusing attention.

Major depression is a multifaceted disorder characterized by primarily by dysphoric mood and loss of interest or pleasure in activities that were once enjoyable. Other physical and psychological symptoms include inability to concentrate, motor disturbances (psychomotor retardation or agitation), feelings of worthlessness, inappropriate guilt, thoughts of suicide, and disturbances in appetite and sleep.

Anxiety disorders are a group of syndromes that include generalized anxiety disorder, panic disorder, phobias, obsessive-compulsive disorder, and post traumatic stress disorder. Although each disorder has its own distinct features, all share common symptoms of excessive worrying, intense fears and dread, hypervigilance and/or somatic symptoms, in the absence of a dangerous situation.

Normal sexual function requires, among others, the ability to achieve and maintain penile erection. Major anatomic structures of the penis that are involved in erectile function include the corpus cavernosum, corpus spinosum, and the tunica albuginea (a collagenous sheath that surrounds each corpus). The corpora are composed of a mass of smooth muscle (trabecula) which contains a network of endothelial-lined vessels (lacunar spaces). Penile tumescence and erection is caused by relaxation of the arteries and corporal smooth muscles, while closing emissary veins, leading to increased blood flow into the lacunar network. Central and peripheral innervation contributes to regulation of the erectile response.

Erectile dysfunction (ED) may result from failure to initiate, fill, or store adequate blood volume within the lacunar network of the penis. Depending on the underlying dysfunction, ED may be vasculogenic, neurogenic, endocrinologic, diabetic, psychogenic, or medication-related.

ED affects 10-25% of middle-aged and elderly men, and has a profound impact on the well-being of affected men. It is currently treated using PDE5 inhibitors such as vardenafil, tadalifil, and sildenafil. Intraurethral alpostadil (prostaglandin EI) may be used in patients that fail on oral agents. In addition, vacuum constriction devices (VCD) are a well-established, noninvasive therapy.

Female sexual dysfunction (FSD) is highly prevalent, age-related, and progressive. It affects 30 to 50% of women. FSD denotes a range of medical problems and is categorized according to disorders of (1) desire, (2) arousal,

(3) orgasm and (4) sexual pain, and symptoms include diminished vaginal lubrication, pain and discomfort with intercourse, decreased arousal, and difficulty achieveing orgasm. On a molecular level, vasoactive intestinal peptide (VIP), nitic oxide (NO), and sex hormones such as estrogens and androgens have been suggested to be important in female sexual function. Current treatment approaches include estrogen replacement therapy, methyl testosterone, PDE5 inhibitors such as sildenafil, the NO-donor L-arginine, prostaglandin EI, phentolamine, and the dopamine agonists apomorphine.

Also preferred is the use of the amorphous phase of (R)-N-piperidinyl-5-(4-chlorophenyl)-1-(2,4-dichlorophenyl)-4,5-dihydro-1H-pyrazole-3-carboxamide according to the invention, and optionally one or more pharmaceutically acceptable excipients, for the preparation of a medicament for the prophylaxis and/or treatment of metabolic syndrome, especially weight independent, cardiovascular diseases, cardiovascular risk factors, glucose intolerance and insulin resistance; for the prophylaxis and/or treatment and/or influencing of dyslipidaemia; for the prophylaxis and/or treatment and/or influencing of blood parameters especially lipid parameters, seeming to lower LDL bodies while increasing HDL bodies, including also the treatment of food disorders (obesity) under conditions of developed diabetes, especially Type II.

The metabolic syndrome and definitions thereof are described in detail by Eckel et al., The Lancet, Vol. 365 (2005), 1415-1428, included herewith by reference. One of the respective definitions was established by the WHO in 1998 (as described in Alberti et al., Diabet. Med. 1998, 15, pages 539-53, the respective description thereof is herewith incorporated by reference and forms part of the present disclosure). The other, more widely accepted, definition of the metabolic syndrome was established by the Adult Treatment Panel (ATP III) of the US National Cholesterol Education Program (NCEP) in 2001, as described in JAMA 2001; 285; 2486-97, the respective description thereof is herewith incorporated by reference and forms part of the present disclosure.

The metabolic syndrome is characterized by an interaction of several physiological parameters such as triglycerides, lipids, blood pressure, glucose levels and insuline levels. Even though obesity may play a critical role in the development of metabolic syndrome, many of its aspects are weight independent, especially some lipid parameters. Especially the positive influence on the weight independent aspects of the metabolic syndrome (see e.g. Pagotto and Pasquali, The Lancet, Vol. 365 (2005), 1363, 1364, included herewith by reference) like some blood parameters, especially lipid parameters is one of the major and surprising advantages of the inventively used substituted pyrazoline compounds.

Since the amorphous phase of (R)-N-piperidinyl-5-(4-chlorophenyl)-1-(2,4-dichlorophenyl)-4,5-dihydro-1H-pyrazole-3-carboxamide according to the invention also has a beneficial effect on the ratio of low density lipoprotein (LDL) to high density lipoprotein (HDL), i.e. they lower the LDL-levels and/or elevate the HDL levels; they are also useful as coating material or co-coating material in stents in order to prevent restenosis.

The term "treatment" as used in the present application encompasses prevention, amelioration and/or complete recovery from the disease. Said term also includes the prevention, amelioration and/or complete recovery of one or more symptoms associated with the disease.

Furthermore, the amorphous phase of (R)-N-piperidinyl-5-(4-chlorophenyl)-1-(2,4-dichlorophenyl)-4,5-dihydro-1H-pyrazole-3-carboxamide according to the invention is connected to relatively weak Herg channel affinity, thus a low risk of prolongation of the QT-interval is to be expected for the compound.

In summary, the inventively used amorphous phase of (R)-N-piperidinyl-5-(4-chlorophenyl)-1-(2,4-dichlorophenyl)-4,5-dihydro-1H-pyrazole-3-carboxamide according to the invention is distinguished by a broad spectrum of beneficial effects, while at the same time showing relatively little undesired effects, i.e. effects which do not positively contribute to or even interfere with the well being of the patient.

Another aspect of the invention is the use of the amorphous phase of (R)-N-piperidinyl-5-(4-chlorophenyl)-1-(2,4-dichlorophenyl)-4,5-dihydro-1H-pyrazole-3-carboxamideaccording to the invention for the manufacture of a medicament for improvement of cardiovascular and/or metabolic risk factors, such as one or more of the following factors:

- Elevated triglycerides, whereby elevated levels of triglycerides are preferably understood as being > 150 mg/dl,
- Low HDL cholesterol, whereby low levels of HDL cholesterol are preferably understood as being < 40 mg/dl in men and < 50 mg/dl in women,
- Hypertension, whereby hypertension is preferably understood as being > 130/85 mmHg,
- Impaired fasting glucose, whereby impaired fasting glucose levels are preferably understood as being > 110 mg/dl,
- Insulin resistance
- Dyslipidemia.

Another aspect of the invention is the use of the amorphous phase of (R)-N-piperidinyl-5-(4-chlorophenyl)-1-(2,4-dichlorophenyl)-4,5-dihydro-1H-pyrazole-3-carboxamide according to the invention for the manufacture of a medicament for the treatment of the weight independent aspects of metabolic syndrome.

Another aspect of the invention is the use of the amorphous phase of (R)-N-piperidinyl-5-(4-chlorophenyl)-1-(2,4-dichlorophenyl)-4,5-dihydro-1H-pyrazole-3-carboxamide according to the invention for the manufacture of a medicament for the treatment of dyslipidemia.

Another aspect of the invention is a method for improving cardiovascular and/or metabolic risk factors, such as one or more of the following factors:

- Elevated triglycerides, whereby elevated levels of triglycerides are preferably understood as being > 150 mg/dl,
- Low HDL cholesterol, whereby low levels of HDL cholesterol are preferably understood as being < 40 mg/dl in men and < 50 mg/dl in women,
- Hypertension, whereby hypertension is preferably understood as being > 130/85 mmHg,
- Impaired fasting glucose, whereby impaired fasting glucose levels are preferably understood as being > 110 mg/dl,
- Insulin resistance
- Dyslipidemia,

in a subject, preferably a human.

Another preferred aspect of the invention is also a method of treatment encompassing all the abovementioned uses, wherein the amorphous phase of (R)-N-piperidinyl-5-(4-chlorophenyl)-1-(2,4-dichlorophenyl)-4,5-dihydro-1H-pyrazole-3-carboxamide according to the invention, is applied to a person in need thereof, treating metabolic syndrome, especially weight independent, cardiovascular diseases especially fighting cardiovascular risk factors, influencing the blood parameters, especially the lipid parameters, diabetes, especially type II, glucose intolerance and insulin resistance, bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit; alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction; cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer; food intake disorders, preferably bulimia, anorexia, cachexia, obesity and/or type II diabetus mellitus (non-insuline dependent diabetes mellitus), more preferably obesity; psychosis; disorders of the central nervous system, disorders of the immune system, disorders of the cardiovascular system, disorders of the endocrinous system, disorders of the respiratory system, disorders of the gastrointestinal tract or reproductive disorders.

The medicament according to the present invention may be in any form suitable for the application to humans and/or animals, preferably humans including infants, children and adults and can be produced by standard procedures known to those skilled in the art. The composition of the medicament may vary depending on the route of administration.

The medicament of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, capsules, lozenges, aqueous or oily solutions, suspensions, emulsions, or dry powdered forms suitable for reconstitution with water or other suitable liquid medium before use, for immediate or retarded release.

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from1 to 2000, preferably 1 to 1500, more preferably 1 to 1000 milligrams or also 20 to 200 mg of active substance to be administered during one or several intakes per day.

**Pharmacological Methods:**

**I. In-vitro determination of affinity to CB1/CB2-Receptors**

a)

The in-vitro determination of the affinity of the inventive substituted pyrazoline compounds to $CB_1/CB_2$-Rezeptors is carried out as described in the publication of Ruth A. Ross, Heather C. Brockie et al., "Agonist-inverse agonist characterization at CB1 and CB2 cannabinoid receptors of L-759633, L759656 and AM630", British Journal of Pharmacology, 126, 665-672, (1999), whereby the transfected human $CB_1$ and $CB_2$ receptors of Receptor Biology, Inc. are used. The radioligand used for both receptors is $[^3H]$-CP55940. The respective parts of the description is hereby incorporated by reference and forms part of the present disclosure.

b)

**Rat cerebellum CB1 binding**

Binding affinity to CB1 receptor was evaluated according to a modification of the method described by Govaerts et al., Eur J Pharmac Sci 23, 233-243 (2004). The respective parts of the description is hereby incorporated by reference and forms part of the present disclosure.

Briefly, cerebellum from male wistar rats (250-300g) were carefully dissected on ice and homogenates were prepared with a Potter-Helveheim homogeniser a cold 50 mM Tris-HCl solution containing 5 mM $MgCl_2$, 1 mM EDTA and 0.25 M sucrose, pH 7.4. The suspension was centrifuged at 1,000 x g for 5 minutes. The supernatants were collected and centrifuged 50,000 x g for 15 minutes. The resulting pellets were then resuspended in Tris-HCl buffer without sucrose, homogenized and incubated for 15 min at 37°C in an orbital shaker bath and centrifuged again at 50,000 x g for 15 min. Pellets were weighted, resuspended in Tris-HCl buffer without sucrose, homogenized with an Ultraturrax homogeniser at 13,500 rpm for 3 x 5 seconds and aliquoted in 0.9 ml volumes in Eppendorf tubes. Alicuotes were centrifuged at 20,800 x g for 5 minutes, supernatants discarded and pellets were frozen at - 80°C until use. Total protein concentration was determined using the Bio-Rad commercial assay based on the Lowry method.
Competitive binding experiments were performed in presence of 1 nM $[^3H]$-CP 55,940 in siliconized glass tubes containing 100 μg protein/tube resuspended in 1ml final volume of 50 mM Tris-HCl, 5 mM $MgCl_2$, 1 mM EDTA, 0.5% (w/v) bovine serum albumin, pH 7.4. Compounds were present at various concentrations and the non specific binding was determined in the presence of 10 μM HU-210. After 1 hour incubation at 30°C, the suspension was rapidly filtered through 0.5% PEI pre-treated GF/B fiber filters on a 96-well harvester and washed 3 times with 3 ml ice-cold binding buffer without bovine serum albumin. Radioactivity on filters was measured with Wallac Winspectral 1414 counter by liquid scintillation in 6 ml Ecoscint H (National Diagnostics, U.K.). Assays were made in triplicates.

Binding data were analyzed by non-linear regression with the software GraphPad Prism Version 3.03.

**II. In-vivo bioassay system for determination of cannabinoid activity**

**Mouse tetrad model**

Substances with affinity for cannabinoid receptors are known to produce a wide range of pharmacological effects. It is also known that intravenous administration of a substance with affinity for cannabinoid receptors in mice produces analgesia , hypothermia, sedation and catalepsy. Individually, none of these effects can be considered as proof that a tested substance has affinity for cannabinoid-receptors, since all of these effects are common for various classes of centrally active agents. However, substances, which show all of these effects, i.e. substances that are active in this so-called tetrad model are considered to have affinity for the cannabinoid receptors. It has further been shown that cannabinoid receptor antagonists are highly effective in blocking the effects of a cannabinoid agonist in the mouse tetrad model.

The tetrad model is described, for example, in the publication of A. C. Howlett et al, International Union of Pharmacology XXVII. Classification of Cannabinoid Receptors, Pharmacol Rev 54, 161-202 , 2002 and David R. Compton et al., "In-vivo Characterization of a Specific Cannabinoid Receptor Antagonist (SR141716A) :Inhibition of Tetrahydrocannbinol- induced Responses and Apparent Agonist Activity", J. Pharmacol. Exp. Ther. 277 , 2, 586-594, 1996. The corresponding parts of the description are hereby incorporated by reference.

**Material and Methods**

Male NMRI mice with a weight of 20-30 g (Harlan, Barcelona, Spain) are used in all of the following experiments.
Before testing in the behavioural procedures given below, mice are acclimatised to the experimental setting. Pre-treatment control values are determined for analgesia hot plate latency (in seconds), rectal temperature, sedation and catalepsy.
In order to determine the agonistic activity of the substance to be tested, the mice are injected intravenously with the substance to be tested or the vehicle alone. 15 minutes after injection, latency in hot plate analgesia is measured. Rectal temperature, sedation and catalepsy are measured 20 minutes after injection.
In order to determine the antagonistic activity the identical procedure is used as for the determination of the agonistic effects, but with the difference that the substance to be evaluated for its antagonistic activity is injected 5 minutes before the intravenous injection of 1.25 mg/kg Win-55,212 a known cannabinoid-receptor agonist.

**Hot plate analgesia**

The hot plate analgesia is determined according to the method described in Woolfe D. et al. "The evaluation of analgesic action of pethidine hydrochloride (Demerol)", J. Pharmacol. Exp. Ther. 80, 300-307,1944. The respective description is hereby incorporated by reference and forms part of the present disclosure.
The mice are placed on a hot plate (Harvard Analgesimeter) at 55 $\pm$ 0.5 °C until they show a painful sensation by licking their paws or jumping and the time for these sensations to occur is recorded. This reading is considered the basal value (B). The maximum time limit the mice are allowed to remain on the hot plate in absence of any painful response is 40 seconds in order to prevent skin damage. This period is called the cut-off time (PC).
Fifteen minutes after the administration of the substance to be tested, the mice are again placed on the hot plate and the afore described procedure is repeated. This period is called the post-treatment reading (PT).
The degree of analgesia is calculated from the formula :

$$\%\ \text{MPE of Analgesia} = (PT - B) / (PC - B) \times 100$$

MPE = Maximum possible effect.

**Determination of sedation and ataxia**

Sedation and ataxia is determined according to the method described in Desmet L. K. C. et al. "Anticonvulsive properties of Cinarizine and Flunarizine in Rats and Mice", Arzneim. - Forsch. (Frug Res) 25, 9, 1975. The respective description is hereby incorporated by reference and forms part of the present disclosure.
The chosen scoring system is

0: no ataxia;
1: doubtful;
2: obvious calmness and quiet;
3 pronounced ataxia;

prior to as well as after treatment.
The percentage of sedation is determined according to the formula:

$$\%\ \text{of sedation} = \text{arithmetic mean} / 3 \times 100$$

**Hypothermia:**

Hypothermia is determined according to the method described in David R. Compton et al. "In-vivo Characterization of a Specific Cannabinoid Receptor Antagonist (SR141716A) Inhibition of Tetrahydrocannbinol- induced Responses and Apparent Agonist Activity", J. Pharmacol Exp Ther. 277 , 2, 586-594, 1996. The respective description is hereby incorporated by reference and forms part of the present disclosure.
The base-line rectal temperatures are determined with a thermometer (Yello Springs Instruments Co., Panlabs)

and a thermistor probe inserted to 25mm before the administration of the substance to be tested. Rectal temperature is again measured 20 minutes after the administration of the substances to be tested. The temperature difference is calculated for each animal, whereby differences of ≥ -2 °C are considered to represent activity.

**Catalepsy:**

Catalepsy is determined according to the method described in Alpermann H. G. et al. "Pharmacological effects of Hoe 249: A new potential antidepressant", Drugs Dev. Res. 25, 267-282. 1992. The respective description is hereby incorporated by reference and forms part of the present disclosure.

The cataleptic effect of the substance to be tested is evaluated according to the duration of catalepsy, whereby the animals are placed head downwards with their kinlegs upon the top of the wooden block.

The chosen scoring system is:

Catalepsy for:

more than 60 seconds = 6; 50 -60 seconds = 5, 40-50 seconds = 4, 30-40 seconds = 3, 20-30 seconds = 2, 5-10 seconds = 1, and less than 5 seconds =0.

The percentage of catalepsy is determined according to the following formula:

$$\% \text{ Catalepsy} = \text{arithmetic mean} / 6 \times 100$$

**III. In vivo testing for antiobesic activity**

a) Accute Treatment

Normally handled rats are habituated to a reversed cycle 12/12h, and the tested compound as well as saline was acutely orally administered. After administration the cumulated food intake (g) is measured at 6 h and 24 h. Following that the difference in body weight between control and compound treated animals is measured. This is a variation of the test according to Colombo et al. as described below.

b) Long-term Treatment

The in-vivo testing for antiobesic activity of the inventive pyrazoline compounds is carried out as described in the publication of G. Colombo et al., "Appetite Suppression and Weight Loss after the Cannabinoid Antagonist SR 141716"; Life Sciences, 63 (8), 113-117, (1998). The respective part of the description is hereby incorporated by reference and forms part of the present disclosure.

**IV. In vivo testing for antidepressant activity**

The in-vivo testing for antidepressant activity of the inventive pyrazoline compounds in the water despair test is carried out as described in the publication of E.T. Tzavara et al., "The CB1 receptor antagonist SR141716A selectively increases monoaminergic neurotransmission in the medial prefrontal cortex: implications for therapeutic actions"; Br. J. Pharmacol. 2003, 138(4):544:53. The respective part of the description is hereby incorporated by reference and forms part of the present disclosure.

**V. Determination of the effect of continued administration of the inventive compounds on body weight in rats**

The weight of the animals was measured for 36 days, and the data grouped into 3 periods for analysis: Period 1, days 1-8 (period for adaptation of the animals to the environmental conditions: one week without treatment plus first day of treatment); Period 2, days 9-22 (treatment period: two weeks); Period 3, days 23-36 (escape treatment period: two weeks). The administration of the drugs started on day 8, immediately after the animal was weighted, and finished on day 21. The treatment period ranges between one day after first administration and one day after last administration. The drugs were administered i.p. once daily. For comparative purposes, absolute weights were transformed into relative percentages (each weight was divided by that of day 8 and multiplied by 100). Three treatment groups were constructed: vehicle, racemic N-piperidinyl-5-(4-chlorophenyl)-1-(2,4-dichlorophenyl)-4,5-dihydro-1H-pyrazole-3-carboxamide (10

mg/kg), and (R)-N-piperidinyl-5-(4-chlorophenyl)-1-(2,4-dichlorophenyl)-4,5-dihydro-1H-pyrazole-3-carboxamide (10 mg/kg).

***VI. In vitro determination of antagonism to CB1-receptor***

**Membrane preparation:**

Chinese hamster ovary (CHO) cells stably expressing recombinant human cannabinoid 1 receptor (CB1) are cultured in nutrient mixture Ham's F 12 supplemented with 10 % heat-inactivated fetal bovine serum, 2 mM L-glutamine, 50 U/ml penicillin, 50 U/ml streptomycin and 0.5 mg/ml geneticin. In order to obtain cells, culture flasks are washed twice with phosphate buffered saline and scraped. Then, cells are collected by centrifugation (200 x g, 10 min) and stored dry at -80˚C. Cells are homogenized in ice-cold 20 mM HEPES, 10 mM EDTA (pH 7.5) and centrifuged at 40,000 x g for 15 min at 4˚C. The membrane pellet is resuspended in 20 mM HEPES, 0.1 mM EDTA (pH 7.5) and centrifuged for 15 min at 4˚C. The final membrane pellet is resuspended in 20 mM HEPES, 0.1 mM EDTA (pH 7.5), and divided in aliquots and stored at -80˚C until use.

**[$^{35}$S]GTPγS binding assay:**

The reaction is performed in 96-well plates. Membranes (15 μg protein/well) are incubated for 60 min at 30 ˚C in buffer (50 mM HEPES, 100 mM KCl, 5 mM $MgCl_2$,1 mM EDTA, 0.1 % wt/vol bovine serum albumin, 5 μM GDP, saponin (10 μg/ml), 0.5 nM [$^{35}$S]GTPγS, pH 7.4) with compound at a final concentration of 1 μM in either the absence or presence of agonist WIN 55,212-2 between 3 nM and 3 μM. The incubation is terminated by rapid filtration through Millipore Multiscreen glass fiber FB, and rinsed two-times with ice-cold assay buffer. Filter plates are dried and 30 μl of scintillation liquid is added. Radioactivity is determined using a Wallac Microbeta Trilux. Each experiment is performed at least in duplicate. A WIN 55,212-2 dose-response experiment either alone or in the presence of Rimonabant (1 μM) is systematically performed.

**Calculations:**

The average of basal [$^{35}$S]GTPγS binding is subtracted from all binding data. In order to compare the antagonism results from one screening campaign to another one, the difference between the maximal agonist effect of WIN 55,212-2 alone, and the maximal antagonism effect due to WIN 55,212-2 plus Rimonabant (1 μM) is defined as 100 %.

**Further methods:**

**Alcohol Intake**

The following protocol may be used to evaluate the effects of alcohol intake in alcohol preferring (P) female rats (e.g. bred at Indiana University) with an extensive drinking history. The following reference provides detailed a description of P rats: Lumeng, L, et al.,"Different sensitivities to ethanol in alcohol-preferring and-nonpreferring rats,"Pharmacol, Biochem Behav., 16, 125-130 (1982).

Female rats are given 2 hours of access to alcohol (10% v/v and water, 2-bottle choice) daily at the onset of the dark cycle. The rats are maintained on a reverse cycle to facilitate experimenter interactions. The animals are initially assigned to four groups equated for alcohol intakes: Group 1-vehicle; Group 2-positive control (e. g. 5.6 mg/kg AM251; Group3-low dose test compound; and Group 4-high dose of test compound. Test compounds are generally mixed into a vehicle of 30% (w/v) -cyclodextrin in distilled water at a volume of 1-2 ml/kg. Vehicle injections are given to all groups for the first two days of the experiment. This is followed by 2 days of drug injections (to the appropriate groups) and a final day of vehicle injections. On the drug injection days, drugs are given sc 30 minutes prior to a 2-hour alcohol access period. Alcohol intake for all animals is measured during the test period and a comparison is made between drug and vehicle-treated animals to determine effects of the compounds on alcohol drinking behavior.

Additional drinking studies can be done utilizing female C57Bl/6 mice (Charles River). Several studies have shown that this strain of mice will readily consume alcohol with little to no manipulation required (Middaugh et al.,"Ethanol Consumption by C57BU6 Mice : Influence of Gender and Procedural Variables" Alcohol, 17 (3),175-183, 1999; Le et al.,"Alcohol Consumption by C57BU6, BALA/c, and DBA/2 Mice in a Limited AccessParadigm" PharmacologyBiochemistry and Behavior,47, 375-378,1994).

For example, upon arrival mice are individually housed and given unlimited access to powdered rat chow, water and a 10 % (w/v) alcohol solution. After 2-3 weeks of unlimited access, water is restricted for 20 hours and alcohol is restricted to only 2 hours access daily. This is done in a manner that the access period was the last 2 hours of the

dark part of the light cycle.

Once drinking behavior is stabilized, testing can commence. Mice are considered stable when the average alcohol consumption for 3 days is 20% of the average for all 3 days. Day 1 of test consists of all mice receiving vehicle injection (sc or ip). Thirty to 120 minutes post injection access is given to alcohol and water. Alcohol consumption for that day is calculated (g/kg) and groups are assigned so that all groups have equivocal alcohol intake. On day 2 and 3, mice are injected with vehicle or drug and the same protocol as the previous day is followed. Day 4 is wash out and no injections are given. Data is analyzed using repeated measures ANOVA. Change in water or alcohol consumption is compared back to vehicle for each day of the test. Positive results would be interpreted as a compound that was able to significantly reduce alcohol consumption while having no effect on water

**Oxygen Consumption Methods:**

Whole body oxygen consumption is measured using an indirect calorimeter (Oxymax from Columbus Instruments, Columbus, OH) in male Sprague Dawley rats (if another rat strain or female rats are used, it will be specified). Rats (e.g. 300-380 g body weight) are placed in the calorimeter chambers and the chambers are placed in activity monitors. These studies are done during the light cycle. Prior to the measurement of oxygen consumption, the rats are fed standard chow ad libitum. During the measurement of oxygen consumption, food is not available. Basal pre-dose oxygen consumption and ambulatory activity are measured every 10 minutes for 2.5 to 3 hours. At the end of the basal pre-dosing period, the chambers are opened and the animals are administered a single dose of compound (the usual dose range is 0.001 to 10 mg/kg) by oral gavage (or other route of administration as specified, i. e. , sc, ip, iv). Drugs are prepared in methylcellulose, water or other specified vehicle (examples include PEG400, 30% beta-cyclo dextran and propylene glycol). Oxygen consumption and ambulatory activity are measured every 10 minutes for an additional 1-6 hours post-dosing.

The Oxymax calorimeter software calculates the oxygen consumption (ml/kg/h) based on the flow rate of air through the chambers and difference in oxygen content at inlet and output ports. The activity monitors have 15 infrared light beams spaced one inch apart on each axis, ambulatory activity is recorded when two consecutive beams are broken and the results are recorded as counts.

Resting oxygen consumption, during pre-and post-dosing, is calculated by averaging the 10-min02 consumption values, excluding periods of high ambulatory activity (ambulatory activity count > 100) and excluding the first 5 values of the pre-dose period and the first value from the post-dose period. Change in oxygen consumption is reported as percent and is calculated by dividing the post-dosing resting oxygen consumption by the pre-dose oxygen consumption *100. Experiments will typically be done with n = 4-6 rats and results reported are mean +/-SEM.

Interpretation :

An increase in oxygen consumption of > 10% is considered a positive result. Historically, vehicle-treated rats have no change in oxygen consumption from pre-dose basal.

**Nicotine Dependence**

An intravenous nicotine self-administration model or place preference model may be used to assess the effects of a test compound on nicotine dependence (see, e.g., Vastola, et al. Physiol. Behav. 77:107-114, 2002; Brower, et al., Brain Res. 930:12-20, 2002).

**Place Preference**

Sprague-Dawley rats are used in this study (Vastola, et al., 2002). Animals are housed in a temperature-controlled, 12h/12h illumination cycle with ad libitum access to food and water. Conditioning and testing are conducted in a chamber divided into two compartments with a door separating the two compartments. Behavior of the animals is recorded by video camera.

Animals are habituated to the injection procedure for several days. The animals are then placed into the test chamber and given free access to both compartments. The initial preference for a particular compartment is determined. For the conditioning trials, animals are injected with nicotine and restricted to the nonpreferred compartment, or the animals are injected with saline and restricted to the preferred compartment. On test day, the door separating the compartments is removed, the animal is placed in the center of the chamber and allowed to move freely between compartments. Time spent in each compartment is scored. Preferential occupancy of the nicotine compartment follows from the conditioned reinforcing effects of nicotine.

### Self-administration

Self-administration in animals is a predictor of a compound's abuse potential in humans. Modifications to this procedure may also be used to identify compounds that prevent or block the reinforcing properties of drugs that have abuse potential. A compound that extinguishes the self- administration of a drug may prevent that drug's abuse or its dependence.

Sprague-Dawley rats are used in this study. Initially, animals are housed in a temperature-controlled, 12h/12h illumination cycle with ad libitum access to food and water. The animals are then implanted with jugular catheters which exit through the animal's back, and each animal is placed in an individual operant chamber (Brower, et al., 2002). The catheters are connected to a computer-driven syringe pump which is located outside of the chamber. The chamber contains two levers with a green light located above each lever. The light is illuminated when nicotine is available.

In a self-administration test, animals are placed in the operant chambers and the levers are randomly designated as an active and inactive lever. Each response on the active lever produces an infusion of nicotine. Presses on the inactive lever have no effect, but are also recorded. Animals are then trained to self -administer nicotine over a set period of time by having drug access during each daily session. Illumination of the chamber house light signals the beginning of the session and the availability of nicotine. When the session ends, the house light is turned off. Initially, a nicotine infusion occurs with every press of the active lever. Once lever-pressing behavior has been established, the number of presses to produce a nicotine infusion is increased. After stable nicotine self-administration is obtained, the effect of a test compound on the nicotine- reinforced behavior may be evaluated. Administration of this test compound prior to the session can either potentiate, extinguish, or produce no change to the self-administrating behavior. Tests are conducted every two days, and the order of the administration of the test compound doses is controlled.

## ALZHEIMER/DEMENTIA EXPERIMENTS

### Morris Water Maze Task

The Morris water maze is a behavioral in vivo test to measure spatial orientation learning and memory through a complex learning task. It is highly suitable for testing compounds that enhance learning and memory. A circular water tank or pool (diameter 2 m, height 0.7 m) is filled with water, and a 10 $cm^2$ platform is placed 1-1.5 cm below the water surface at a defined location within the pool. The escape platform is not visible for an animal swimming in the water tank. For the experiment, a rat or mouse is placed into the pool to swim freely.

The animals have the task to localize the submerged platform, and the time and distance required for successful retrieval is measured. Multiple extra-maze cues are provided by the furniture in the room, including desks, computer equipment, a second water tank, the presence of the experimenter, and by a radio on a shelf that is playing softly.

Before administration of the test compound, animals are usually trained in the task 4 times a day for 5 days. Test compounds are administered orally or intraperitoneally on the day of the experiment at a defined time (e.g., 30 minutes before the first swim test). Control animals are dosed with the corresponding vehicle not containing test compound. Active compounds yield shorter times and distances to localize the platform (i.e., the better the animal remembers the location of the platform, the shorter the distance covered and the faster the platform is reached).

The test can also be carried out using transgenic or cognitively impaired animals. Cognitive impairment is induced either by old age or experimentally through brain lesions, such as bilateral lesions of the entorhinal cortex in rats. Such lesions can be induced by intracerebral injections of the excitotoxin ibotenic acid.

### Object Recognition Task

The object recognition task is used to assess the effects of compounds on the cognitive performance of rodents. A rat is placed in an open field, in which two identical objects are located. The rats inspects both objects during the initial trial of the test. After a certain retention interval (e.g., 24 hours), a second trial is carried out. Here, one of the two objects used in the first trial (the 'familiar' object) and a novel object are placed in the open field, and the inspection time at each of the objects is measured. Good retention is reflected by higher exploration times towards the novel compared with the 'familiar' object.

Administration of the putative cognition enhancer prior to the first trial predominantly allows assessment of the effects on acquisition, and on the consolidation processes. Administration of the test compound after the first trial allows to assess the effects on consolidation processes, whereas administration before the second trial allows to measure effects on retrieval processes.

**Passive Avoidance Task**

The passive avoidance task assesses memory performance in rats and mice. The inhibitory avoidance uses an apparatus consisting of a box with two compartments separated by a guillotine door that can be operated by the experimenter. One compartment is illuminated with bright light, and the other compartment is dark. A threshold of 2 cm separates the two compartments when the guillotine door is raised. When the door is open, the illumination in the dark compartment is about 2 lux. The light intensity is about 500 lux at the center of the floor of the light compartment.

Two habituation sessions, one shock session, and a retention session are given, separated by inter-session intervals of 24 hours. During the habituation sessions and the retention session, the rat is allowed to explore the apparatus for 300 seconds. The rat is placed in the light compartment, facing the wall opposite to the guillotine door. After an accommodation period of 15 seconds, the guillotine door is opened so that all parts of the apparatus can be visited freely. Rats normally avoid brightly lit areas and will enter the dark compartment within a few seconds.

In the shock session, the guillotine door between the compartments is lowered as soon as the rat has entered the dark compartment with all paws, and a scrambled 1 mA footshock is administered for 2 seconds. Then the rat is removed from the apparatus and returned into its home cage. The procedure during the retention session is identical to that of the habituation sessions.

The step-through latency, that is, the first latency of entering the dark compartment (in seconds) during the retention session is an index of the memory performance of the animal:

a better retention is assumed if the latency to enter the dark compartment is longer. A test compound is given 30 minutes before the shock session, together with 1 mg/kg scopolamine. Scopolamine impairs the memory performance during the retention session 24 hours later. If the test compound increases the enter latency compared with the scopolamine-treated controls, it is considered to possess cognition enhancing activity. T-maze Spontaneous Alternation Task

The T-maze spontaneous alternation task (TeMCAT) assesses the spatial memory performance in mice. The start arm and the two goal arms of the T-maze are provided with guillotine doors that can be operated manually by the experimenter. A mouse is put into the start arm at the beginning of training. In the first trial, either the left or right goal arm is blocked by lowering the respective guillotine door (forced trial).

After the mouse has been released from the start arm, it will explore the maze, eventually entering the open goal arm, and return to the start position, where it will be confined for 5 seconds, by lowering the guillotine door. Then, the animal can choose freely between the left and right goal arm (all guillotine-doors opened) during 14 additional trials (free choice trials). As soon as a mouse has entered one goal arm, the other arm is closed. The mouse eventually returns to the start arm and is free to visit whichever arm it wants after having been confined to the start arm for 5 seconds. After completion of 14 free choice trials in one session, the animal is removed from the maze.

Out of the 14 trials the alternations in percent are calculated. This percentage and the total time needed to complete the first forced trial and the subsequent 14 free choice trials (in seconds) is analyzed. In addition, cognitive deficits can be induced by injection of scopolamine 30 minutes before the start of the training session. A cognition enhancer, administered before the training session, will at least partially, antagonize the scopolamine-induced reduction in the spontaneous alternation rate.

**Depression Model**

A forced swim or tail suspension model may be used to assess the efficacy of antidepressant compounds (see , e.g., Porsolt, et al., Nature 266:730-732, 1977; Stem, et al., Psychopharmacology 85:367-370, 1985).

**Forced Swim Test**

Rats or mice are placed in a cylinder filled with water 23-25 ˚C from which no escape is possible. Initially, animals struggle and try to escape, but eventually adopt a characteristic immobile posture and make no further attempts to escape except for small movements needed their head above water. Animals are dosed with a compound and the activity (swimming or climbing) or immobility is measured by an observer. The immobility is considered by some to reflect a 'behavioral despair' in which animals cease to struggle to escape the aversive situation. A wide variety of clinically used antidepressants (TCAs, MAOIs, SSRIs, atypicals) decrease immobility in this test and has a good predictive validity in that it detects antidepressants with different mechanisms of action but its construct validity is weak. At least two distinct active behavioral patterns are produced by pharmacologically selective antidepressant drugs. Serotonin-selective reuptake inhibitors increase swimming behavior, whereas drugs acting primarily to increase extracellular levels of norepinephrine or dopamine increase climbing behavior. There are false positives (psychostimulants) but relatively few false

negatives ([beta]-adrenergic agonists). The test is sensitive to muscle-relaxant (benzodiazepines) and sedative (neuroleptics) effects, leading to enhanced immobility. False positives and false negatives can often be screened by measuring if the compound produces locomotor stimulation or sedation.

### Tail Suspension Test

When suspended by the tail, mice will initially struggle and try to escape and then alternate between active escape attempts and immobililty. In this test, animals are dosed with a compound and the immobility is measured by an observer for 6 min. Porsolt describes the immobile behavior as 'behavioral despair' which animals cease to struggle to escape the aversive situation. A large variety of clinically antidepressants (tricyclics, MAOIs, SSRIs, and atypicals) reduce immobility in this model. The test has a good predictive validity for antidepressant activity and works for most antidepressant classes including but has some false positives (psychostimulants). The test is sensitive to muscle-relaxant (benzodiazepines) and sedative (neuroleptics) effects, which lead to enhanced immobility. False positives and false negatives can often be screened by measuring if the compound produces locomotor stimulation or sedation. Strain differences in the tail suspension test have been found in mice. The tail suspension test has some face validity but its construct validity is rather weak.

### Schizophrenia Model

A prepulse inhibition model may be used to assess the efficacy of antipsychotic compounds (see Swerdlow and Geyer, Schizophrenia Bulletin 24: 285-301, 1998).

### Prepulse Inhibition

Prepulse inhibition is the process whereby a relatively mild stimulus, the prepulse, suppresses the response to a strong, startle-eliciting stimulus when the prepulse precedes the startle stimulus by a brief duration (about 10 to 500 milliseconds). Prepulse inhibition is a cross-species phenomenon (ie, it is present in mammals ranging from mice to humans), yet it is relatively absent among schizophrenic patients. The deficit in PPI in schizophrenic patients is thought to reflect the loss of sensorimotor gating that may lead to sensory flooding and cognitive fragmentation. In this test, mice or rats are administered compounds and individually placed into a holder on a transducer platform to measure whole body startle. The holder is housed in a startle chamber with background white noise. Following a brief habituation period, animals are given multiple trials of a weak auditory prepulse stimululs, followed by a strong auditory startle stimulus. Four types of trials are given: prepulse plus startle, prepulse alone, startle alone, and no stimulation. PPI is measured as the amount of inhibition of startle following the prepulse and is expressed as the percentage of basic startle. As a control, measurements are taken in the no stimulation and prepulse alone trials. PPI is considered a test with good predictive, face and construct validity for schizophrenia. Putative antipsychotics can be tested alone to determine if they enhance PPI. Alternately, antipsychotics can be screened to determine if they block various agents that disrupt PPI (apomorphine, d- amphetamine, PCP, ketamine, DOI). Finally, mutant mice with or without drugs can be screened using the PPI procedure.

### Anxiety Model

An elevated plus maze model may be used to assess the efficacy of anxiolytic compounds (see Pellow and File, Pharm. Biochem. Behav. 24, 525-529, 1986).

### Elevated Plus Maze

The elevated plus maze is widely used as an anxiety paradigm that examines the conflict between the drive to explore and the aversiveness of heights and open spaces of rats or mice. The maze is a cross made up of two open and two closed arms that is raised above the ground. The combination of light, the open arms, and the height is thought to produce unconditioned fear or anxiety responses in mice or rats. The test apparatus is an open top maze constructed of opaque plastic with alternating open and enclosed arms. For rats, each arm is 45-55 cm long and 8-12 cm wide, with the sides of the enclosed arms 35-45 cm high, the juncture approximately 10 x 10 cm, and the maze is elevated 45-55 cm above the floor. The mouse elevated plus maze consists of two closed arms (15 x 6 x 30 cm) and two open arms (1 x 6 x 30 cm) forming a cross, with a quadrangular center (6 x 6cm). The maze is placed 50 cm above the floor. Testing is performed in a room free of noise and distraction. On test days animals are administered drug or vehicle. If a pretreatment period is necessary, the animals are returned to the home cage for the duration of the pretreatment time; otherwise, the animals are placed in a clear plastic holding chamber singly or with cage mates for 1-10 minutes prior to

test time. Rats are then placed in the center of the maze always oriented in the same direction, either consistently facing an open arm or an enclosed arm. For 5-10 minutes, entries into each arm and the time spent in each arm are recorded by the observer(s) or by videotape or a computer receiving input from a video camera mounted above the maze. To count as an entry, all four paws must be inside the arm. If necessary, additional measures of anxiety-related behaviors will be recorded, i.e., time spent motionless, time spent in the center, time spent grooming, and the number of rears, stretching postures or feces produced. Following testing the animals are returned to the home cages. When animals are placed in the center of the maze, they spend most of their time in the closed arms, avoiding the open arms. Anxiolytic drugs, such as benzodiazepines, will increase the amount of time animals spend in the open arms. The test is also sensitive to anxiogenic drugs, which lends strong support for its predictive validity.

**Erectile Dysfunction**

Drugs affecting erectile function may be tested by measuring the effect on apomorphine-evoked increases in intracavernous pressure in the awake rat as described by Andersson, et al., (J. Urol. 161 : 1707-17] 2, 1999). One end of a polyethylene tubing is implanted into the cavernosal space of the penis of male Sprague-Dawley rats. After recovery from the surgery, intracavernous pressure is recorded using a pressure transducer connected to a multichannel pen-recorder. Erections are induced by administration of apomorphine (100-250 ug/kg s.c.) with or without test compound, and the results are compared for the treated group and the non-treated group.

**Female Sexual Dysfunction**

Systems to test compounds for the treatment of female sexual dysfunction include in vitro and in situ models using vaginal or clitoral smooth muscle preparations, histological evaluation, and vaginal blood flow assessments. In vivo studies of sexual responses focus on behavioral paradigms involving lordotic posturing and receptivity, as well as indices of motivation using a dual chamber pacing method (see, e.g., Hale, et al., Int. J. Impot. Res. 15 Suppl 5: S75-79, 2003).

The present invention is illustrated below with the aid of examples. These illustrations are given solely by way of example and do not limit the general spirit of the present invention.

**Examples**:

**Abbreviations:**

| | |
|---|---|
| Eq. | Equivalent |
| Proc. | Process |
| Solv. Cryst. | Solvent used in crystallisation experiment |
| T Cryst. | Temperature at which crystallisation experiment was carried out |
| $1^{st}$ Cryst. | first crystallisatioN |
| $2^{nd}$ Crys. | second crystallisation |
| r.t. | room temperature (≈ 20-25 ˚C) |

**General description:**

Racemic 5-(4-chlorophenyl)-1-(2,4-dichlorophenyl)-4,5-dihydro-pyrazole-3-carboxylic acid is optained as described in international patent applications WO2005/077909 (exemplified in example 0) and WO 2005/077911. The respective descriptions are hereby incorporated by reference and form part of the present disclosure.

The resolution of the racemate of N-piperidinyl-5-(4-chlorophenyl)-1-(2,4-dichlorophenyl)-4,5-dihydro-1H-pyrazole-3-carboxamide may be carried out by methods known to those skilled in the art, e.g. column chromatography leading to Compound A as described below. The racemate of N-piperidinyl-5-(4-chlorophenyl)-1-(2,4-dichlorophenyl)-4,5-dihydro-1H-pyrazole-3-carboxamide is optained as described in international patent application WO 2005/077911. The respective description is hereby incorporated by reference and form part of the present disclosure.

However its intermediate, racemic 5-(4-chlorophenyl)-1-(2,4-dichlorophenyl)-4,5-dihydro-pyrazole-3-carboxylic acid may easily be separated into the respective enantiomers via reaction with a chiral base. The process for this resolution is described below.

**Resolution of the enantiomers of 5-(4-chlorophenyl)-1-(2,4-dichlorophenyl)-4,5-dihydro-pyrazole-3-carboxylic acid**

The resolution of the enantiomers of 5-(4-chlorophenyl)-1-(2,4-dichlorophenyl)-4,5-dihydro-pyrazole-3-carboxylic acid was carried out via reaction with the following chiral bases:

- Brucine
- Quinine
- (-)-Cinchonidine
- (+)-Cinchonine
- R-(+)-1-Phenylethylamine
- (1R, 2S)-(-)-Ephedrine hydrochloride
- (1S,2R)-(+)-Ephedrine hydrochloride.

In each case the reactions were carried out with 0.5 and 1 equivalents of base in respect to 1 equivalent of the acid compound and by using the following solvents

- Ethanol
- Acetone
- Acetonitril
- Dioxane
- Ethylacetate
- Chloroform.

The results are summarized in the following tables. It may be understood that the afore mentioned crystallisation experiments that are not reflected in the following tables did not yield crystals of the respective salts under the given conditions.

However, suitable conditions for crystallization of these salts can be determined by those skilled in the art via routine experiments.

In the following tables

Acid represents racemic 5-(4-chlorophenyl)-1-(2,4-dichlorophenyl)-4,5-dihydro-pyrazole-3-carboxylic acid

R-Acid represents the respective derivative of (R)-5-(4-chiorophenyl)-1-(2,4-dichlorophenyl)-4,5-dihydro-pyrazole-3-carboxylic acid

S-Acid represents the respective derivative of (S)-5-(4-chlorophenyl)-1-(2,4-dichlorophenyl)-4,5-dihydro-pyrazole-3-carboxylic acid

**Processes for crystallisation:**

**Process A:** A solution of the chiral base was added on top of a solution of the racemic acid at room temperature.

**Process C:** A solution of the racemic acid was added on top of a solution of the chiral base. The mixture was heated to reflux and solvent was added until dissolution was complete. The solution was left to crystallisation at r.t.

**Process D:** The chiral base was directly added on top of a solution of the racemic acid at room temperature.

**Process E:** The chiral base was directly added on top of a solution of the racemic acid at reflux temperature.

**Process F:** The solution of the salt was evaporated to dryness. The residue was dissolved in a minimum amount of the solvent under reflux heating. The solution was left to crystallisation at r.t.

**Resolution with Brucine**

| Brucine | | | | | | | | | |
|---|---|---|---|---|---|---|---|---|---|
| 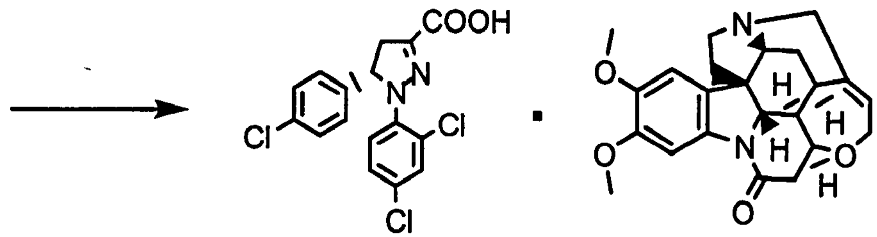 | | | | | | | | | |
| Acid 9 (mmol) | Eq. amine | Proc. | Solvent for crystallisation | T Cryst. | Yield 1st Cryst. % | % S-Acid | % R-Acid | Yield 2nd Cryst. % | % S-Acid |
| 0,4 g (1,09 mmol) | 0,5 | A | 8 ml acetone | r.t. | 44,6 | 98,8 | 1,2 | 5,6% | 99,4 |
| 0,4 g (1,09 mmol) | 0,5 | A | 11,5 ml acetonitrile | r.t. | 50,7 | 47,5 | 52,4 | | |
| 0,4 g (1,09 mmol) | 1 | A | 17 ml acetonitrile | r.t. | 25,6 | 45,7 | 54,3 | | |

**Resolution with Quinine**

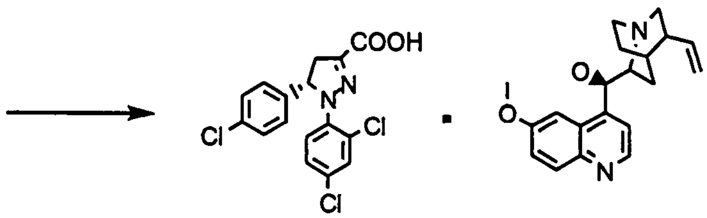

| Acid 9 (mmol) | Eq. amine | Proc. | Solvent for crystallisation | T Crys. | Yield 1st Cryst. % | % S-Acid | % R-Acid |
|---|---|---|---|---|---|---|---|
| 0,4 g (1,09 mmol) | 1 | A | 8 ml dioxane | r.t. | 49,46 | 91,6 | 8,3 |
| 0,4 g (1,09 mmol) | 1 | F | 15ml acetonitrile | r.t. | 26 | 94,4 | 5,5 |
| 0,4 g (1,09 mmol) | 1 | F | 2ml ethylacetate | r.t. | 25 | 96,7 | 3,3 |
| 0,4 g (1,09 mmol) | 0,5 | A | 4ml dioxane | r.t. | 38,5 | 97,5 | 2,5 |

**Resolution with (-)-Cinchonidine**

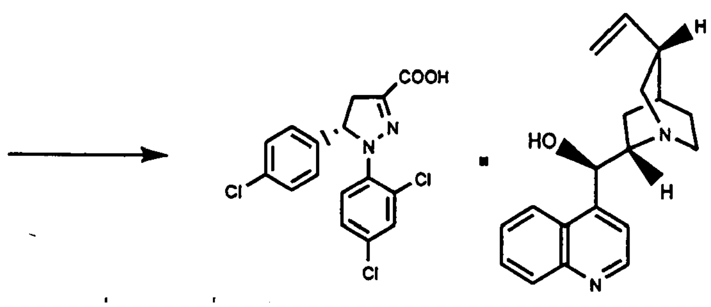

| Acid g (mmol) | Eq. amine | Proc. | Solvent for crystallisation | T Cryst. | Yield 1st Cryst. % | % S-Acid | % R-Acid |
|---|---|---|---|---|---|---|---|
| 0,4 g (1,09 mmol) | 1 | F | 2ml dioxane | r.t. | 31 | 94,4 | 5,6 |
| 0,4 g (1,09 mmol) | 1 | F | 19ml Ethylacetate | r.t. | 28,5 | 95,8 | 4,2 |
| 0,4 g (1,09 mmol) | 1 | F | 20ml acetone | r.t. | 19,6 | 96,9 | 3,1 |
| 0,4 g (1,09 mmol) | 1 | F | 24ml acetonitrile | r.t. | 42 | 85,8 | 14,1 |

**Resolution with (+)-Cinchonine**

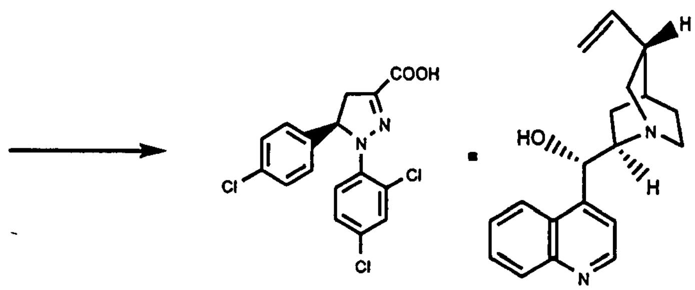

| Acid | Eq. amine | Proc. | Solvent for crystallisation | T Cryst. | Yield 1st Cryst. % | % S-Acid | % R-Acid |
|---|---|---|---|---|---|---|---|
| 0,4 ( 1,09 mmol) | 1 | C | 50 ml acetone | r.t. | 32,8 | 7,42 | 92,57 |
| 0,4 (1,09 mmol) | 0,5 | F | 30ml acetone | r.t. | 23,5 | 4,0 | 95,9 |
| 0,4 (1,09 mmol) | 0,5 | C | 50 ml acetonitrile | r.t. | 31,5 | 3,07 | 96,92 |
| 0,4 (1,09 mmol) | 1 | C | 50 ml acetonitrile | r.t. | 78,25 | 39,01 | 60,98 |
| 0,4 (1,09 mmol) | 0,5 | C | 15 ml ethylacetate | r.t. | 14,9 | 3,62 | 96,37 |
| 0,4 (1,09 mmol) | 1 | C | 27 ml ethylacetate | r.t. | 35 | 7,78 | 92,21 |
| 0,4 (1,09 mmol) | 1 | F | 4ml dioxane | r.t. | 21 | 33,5 | 66,4 |

**Resolution with R-(+)-1-Phenylethylamine**

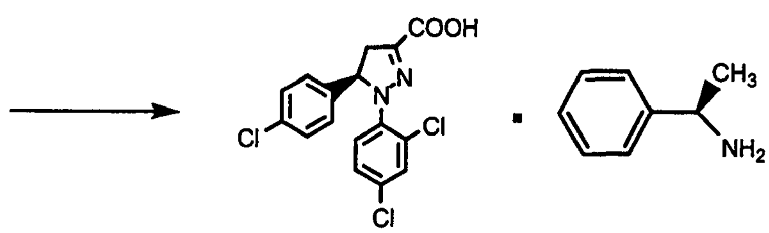

| Acid | Eq. amine | Proc. | Solvent for crystallisation | T Cryst. | Yield 1st Cryst. % | % S-Acid | % R-Acid |
|---|---|---|---|---|---|---|---|
| 0,4 (1,09 mmol) | 1 | D | 1,6ml ethanol | r.t. | 22 | 51,4 | 48,6 |
| 0,4 (1,09 mmol) | 0,5 | E | 6ml acetonitrile | r.t. | 11 | 8,1 | 91,9 |
| 0,4 (1,09 mmol) | 1 | E | 6ml acetonitrile | r.t. | 50 | 36,8 | 63,2 |

(continued)

| Acid | Eq. amine | Proc. | Solvent for crystallisation | T Cryst. | Yield 1st Cryst. % | % S-Acid | % R-Acid |
|---|---|---|---|---|---|---|---|
| 0,4 (1,09 mmol) | 1 | E | 6ml dioxane | ≈5˚C | 5 | 4,6 | 95,3 |

**Detailed Description:**

**Example 1: (R)-N-piperidinyl-5-(4-chlorophenyl)-1-(2,4-dichlorophenyl)-4,5-dihydro-1H-pyrazole-3-carboxamide (Compound A)**

**Resolution of 5-(4-chlorophenyl)-1-(2,4-dichlorophenyl)-4,5-dihydro-pyrazole-3-carboxylic acid with (+)-Cinchonine**

35 g (294.68 mmol) of racemic 5-(4-chlorophenyl)-1-(2,4-dichlorophenyl)-4,5-dihydro-pyrazole-3-carboxylic acid in 630 m acetonitril were added to a suspension of 16.39 g (47.34 mmol) of (+)-Cinchonine in 920 ml acetonitrile under vigorous stirring. The resulting suspension was heated to reflux and acetonitrile (1300 ml) was added until dissolution was complete. The resulting solution was left to crystallise at room temperature over the weekend, whereupon a crystalline solid was obtained. The crystalline solid was filtered off, washed with 50 ml of cold acetonitrile and dried to give 25.26 g of a white solid (ee ≈ 91-92%). No second fraction of crystals could be obtained from the mother liquors, neither upon cooling in the refrigerator nor upon concentration. Thus, recrystallization of the diastereomeric salt was carried out in different solvents in order to improve the enantiomeric excess:

| Solvent | Yield | Ratio of enantiomers % (R) / % (S) |
|---|---|---|
| Acetonitrile | 90 % | 99,6 / 0,4 |
| EtOH-$H_2O$ | 68,5 % | 99,3 / 0,7 |
| Isopropanol | 55 % | 98,9 / 1,1 |

Consequently, the product was recrystallized from acetonitrile to give 22.6 g of crystals (yield 72 mol-% related to chiral base). The ratio of enantiomers determined by capillary electrophoresis was:

99.7 % of (R)-5-(4-chlorophenyl)-1-(2,4-dichlorophenyl)-4,5-dihydro-pyrazole-3-carboxylic acid (+)-Cinchonine
0.3 % of (S)-5-(4-chlorophenyl)-1-(2,4-dichlorophenyl)-4,5-dihydro-pyrazole-3-carboxylic acid (+)-Cinchonine ee= 99.4 %.

**Preparation of (R)-5-(4-chlorophenyl)-1-(2,4-dichlorophenyl)-4,5-dihydro-pyrazole-3-carboxylic acid from addition compound (R)-5-(4-chlorophenyl)-1-(2,4-dichlorophenyl)-4,5-dihydro-pyrazole-3-carboxylic acid-(+)-Cinchonine**

22.59 g (34 mmol) of the diastereomeric salt, which was recrystallized from acetonitrile, were suspended in 200 ml of 6N HCl and stirred at room temperature for 15 minutes. Afterwards, 200 ml of toluene were added until dissolution was complete. The mixture was stirred for 30 minutes and the phases were separated. The aqueous phase was extracted with toluene, the combined organic phases were washed with water, dried over sodium sulfate, filtered and evaporated to dryness, whereupon 11.9 g (95 %) of a white microcrystalline solid (melting point 129-133) were obtained.
Enantiomeric excess determined bY capillary electrophoresis. ee = 99.2 %
Chemical purity determined by HPLC: 99,3 %
$[\alpha]_D$ (c=1,23˚C, MeOH) = -429.

**Isolation of the enantiomer (R)-5-(4-chlorophenyl)-1-(2,4-dichlorophenyl)-4,5-dihydro-pyrazole-3-carboxylic acid from the mother liquors obtained from the crystallisation of the enantiomer (S)-5-(4-chlorophenyl)-1-(2,4-dichlorophenyl)-4,5-dihydro-pyrazole-3-carboxylic acid with Brucine**

The residue obtained from the evaporation of the mother liquors obtained from the crystallisation of (S)-5-(4-chlorophenyl)-1-(2,4-dichlorophenyl)-4,5-dihydro-pyrazole-3-carboxylic acid with Brucine (see below) was treated with 6N HCl as described above for obtaining the free acid. After evaporating the organic phase 21.86 g of a mixture with a theoretical composition of 20 mmoles (S)-5-(4-chlorophenyl)-1-(2,4-dichlorophenyl)-4,5-dihydro-pyrazole-3-carboxylic acid and 39.9 mmoles of (R)-5-(4-chlorophenyl)-1-(2,4-dichlorophenyl)-4,5-dihydro-pyrazole-3-carboxylic acid were obtained. A suspension of 13.82 g (39.9 mmoles) of (+)-cinchonine in 1000 ml of acetonitrile were added and the mixture was heated to reflux. More acetonitrile was added until dissolution was complete (total volume of acetonitrile 4000 ml). The mixture was then slowly cooled to room temperature to obtain crystals. After a few hours a white solid was obtained, which was filtered off and dried to give 22.42 (yield 84,5% related to the salt of (R)-5-(4-chlorophenyl)-1-(2,4-dichlorophenyl)-4,5-dihydro-pyrazole-3-carboxylic acid).
Enantiomeric excess as determined by capillary electrophoresis was ee = 92 %.

The purity of the isolated salt was comparable to the one of the salt obtained directly from the resolution of the racemate. Consequently, the same purification process as described above could be applied.

**Resolution of racemic 5-(4-chlorophenyl)-1-(2,4-dichlorophenyl)-4,5-dihydro-pyrazole-3-carboxylic acid with Brucine**

Preparation of the diastereomeric salt with Brucine

At room temperature 15.74 g (39.9 mmol) of Brucine were dissolved in 475 ml of acetone under vigorous stirring. A solution of 29.5 g (79.8 mmol) of the racemic 5-(4-chlorophenyl)-1-(2,4-dichlorophenyl)-4,5-dihydro-pyrazole-3-carboxylic acid in 85 ml acetone was added. The resulting mixture was allowed to crystallise for a couple of days, whereupon a crystalline solid was obtained. The crystalline solid was filtered off, washed with cold acetone and dried to yield 13.66 g (45 mol % related to the base) of a beige-coloured solid were obtained.

The ratio of the enantiomers as determined by capilar electrophoresis was:

99 % of (S)-5-(4-chlorophenyl)-1-(2,4-dichlorophenyl)-4,5-dihydro-pyrazole-3-carboxylic acid Brucine

1 % of (R)-5-(4-chlorophenyl)-1-(2,4-dichlorophenyl)-4,5-dihydro-pyrazole-3-carboxylic acid Brucine

Enantiomeric excess ee = 98 %

The filtered mother liquors were left in the refrigerator and yielded a second fraction of crystals, which were filtered off and dried to give another 1.56 g (5.1 molar % related to the base).

The analysis of the 2$^{nd}$, fraction via capillar electrophoresis gave
99.3 % of (S)-5-(4-chlorophenyl)-1-(2,4-dichlorophenyl)-4,5-dihydro-pyrazole-3-carboxylic acid Brucine
0.7 % of (R)-5-(4-chlorophenyl)-1-(2,4-dichlorophenyl)-4,5-dihydro-pyrazole-3-carboxylic acid Brucine
Enantiomeric excess ee = 98.6 %
Total yield:

15.22 g (50.1 mol% related to base) of the salt with ee = 98 %.

**Determination of the chirality of the Brucine salt via X-ray crystallography**

The chirality of the enantiomer that crystallized with Brucine (ee 98 %) was determined via x-ray crystallography and corresponds to the S-enantiomer. Accordingly, the other enantiomer that crystallized with (+)-cinchonine is the corresponding R-enantiomer.

**Preparation of (R)-N-piperidinyl-5-(4-chlorophenyl)-1-(2,4-dichlorophenyl)-4,5-dihydro-1H-pyrazole-3-carboxamide (Compound A)**

9.94 g (26.89 mmol) of (R)-5-(4-chlorophenyl)-1-(2,4-dichlorophenyl)-4,5-dihydro-pyrazole-3-carboxylic acid were dissolved in 95 ml of toluene, followed by the addition of 2.35 ml (32.3 mmoles) of thionyl chloride and 4 drops of DMF. Under anhydrous nitrogen atmosphere the mixture was heated to a temperature of 75-80° C under vigorous stirring for two hours and then allowed to cool to room temperature. Said acid chloride solution was then added dropwise to a solution of 3.47 ml (31.2 mmol) of N-Aminopiperidine, 15 ml (107.6 mmol) of triethylamine and 38 ml of anhydrous toluene, which was cooled to 0-5 °C, thereby keeping the temperature below 10 °C during the addition. The reaction mixture was stirred at room temperature overnight, whereupon a suspension was obtained. Water and Toluene were added to said suspension until dissolution was complete and the phases were separated. The aqueous phase was extracted with toluene, the combined organic phases washed with an NaOH solution (10 %) and water, dried over sodium

sulfate, filtered off and evaporated to dryness to yield 13.41 g of light yellow oil, which failed to crystallise. The oil was purified via column chromatography over silica gel (eluent: petrol ether ethylacetate 90: 10 to 60: 40) to give 10.5 g (83.5 %) of (R)-N-piperidinyl-5-(4-chlorophenyl)-1-(2,4-dichlorophenyl)-4,5-dihydro-1H-pyrazole-3-carboxamide in form of an amorphous solid of light yellow colour (melting point 75-78 ˚C).

$^1$H-NMR (DMSO-$d_6$) $\delta$ ppm : 9,25 (s, 1 H), 7,5 (d, J=8,8Hz, 1H), 7,4 (d, J=2,3Hz, 1 H), 7,3-7,25 (2d, J=8,8 and 8,5Hz, 3H), 7,1 (d, J=8,5Hz, 2H), 5,8 (dd, J=5,7 and 11,8 Hz, 1H), 3,65 (dd, J=11,8 and 18,1Hz, 1H), 3,0 (dd, J=5,7 and 18,1Hz, 1H), 2,75 (m, 4H), 1,5 (m, 4H), 1,2 (m, 2H).

Analysis of the enantiomer via chiral HPLC: ee = 100 %

Chemical purity determined by HPLC: 98,5%

$[\alpha]_D$ (c=1, 23˚C, MeOH) = - 293,5

**Example 2:**

**Amorphous phase of (R)-N-piperidinyl-5-(4-chlorophenyl)-1-(2,4-dichlorophenyl)-4,5-dihydro-1H-pyrazole-3-carboxamide**

The amorphous phase is obtained by the following way:

A sample of the compound according to example 1 (Compound A / (R)-N-piperidinyl-5-(4-chlorophenyl)-1-(2,4-dichlorophenyl)-4,5-dihydro-1H-pyrazole-3-carboxamide) is heated to 80˚C (a temperature above the melting point of 75-78 ˚C see above) until it is completely melted. Subsequently it is cooled to room temperature until it is completely solid again to yield the amorphous phase of (R)-N-piperidinyl-5-(4-chlorophenyl)-1-(2,4-dichlorophenyl)-4,5-dihydro-1H-pyrazole-3-carboxamide.

**Example 2b:**

**DSC analysis of the amorphous phase of (R)-N-piperidinyl-5-(4-chlorophenyl)-1-(2,4-dichlorophenyl)-4,5-dihydro-1H-pyrazole-3-carboxamide**

The DSC analysis of the *amorphous phase* presents a base line step between 60 and 70˚C corresponding to the glass transition (Tg). No other peak is observed at higher temperatures, indicating that, at least during the analysis, this phase does not crystallize. Since the Tg is a reversible phenomenon, it can be better measured if first the sample is heated to a temperature above the Tg, is then cooled at the same rate to room temperature, and is again heated above the Tg. The true onset of the Tg for the amorphous phase is at 63-66˚C.

**Example 2b:**

**TG analysis of the amorphous phase of (R)-N-piperidinyl-5-(4-chlorophenyl)-1-(2,4-dichlorophenyl)-4,5-dihydro-1H-pyrazole-3-carboxamide**

In the TG analysis of the *amorphous modification* a weight loss of 1% is observed at temperatures below 100˚C. This could be due to residual solvent in the sample. The second weight step occurs at temperatures higher than 300˚C and it is due to decomposition of the sample.

**Example 2c:**

**FTIR Spectrum of the amorphous phase of (R)-N-piperidinyl-5-(4-chlorophenyl)-1-(2,4-dichlorophenyl)-4,5-dihydro-1H-pyrazole-3-carboxamide**

In the FTIR spectrum the *amorphous phase* can be differentiated from crystalline forms because all peaks in the spectrum are wider and less defined. Specifically, it shows an absence of a peak at 3325 $cm^{-1}$, but it shows some peaks at 1656 $cm^{-1}$ and 1473 $cm^{-1}$ An example spectrum of one sample can be seen in Figure 7.

**Example 2e:**

**FTRaman spectrum of the amorphous phase of (R)-N-piperidinyl-5-(4-chlorophenyl)-1-(2,4-dichlorophenyl)-4,5-dihydro-1H-pyrazole-3-carboxamide**

In the FTRaman spectrum of the *amorphous phase* bands are less sharp than with crystalline forms. Specifically it shows a peak at 1666 ± 5 $cm^{-1}$. An example spectrum of one sample can be seen in Figure 8.

**Example 2f:**

**Standard X-Ray Powder diffraction pattern of the amorphous phase of (R)-N-piperidinyl-5-(4-chlorophenyl)-1-(2,4-dichlorophenyl)-4,5-dihydro-1H-pyrazole-3-carboxamide**

See Figure 7).

**Pharmacological Data**

**A) In-Vitro Data**

The binding of pyrazoline compounds of general formula I to the CB1-receptor was determined as described in the section Pharmacological methods, part I.

The pyrazoline compound A shows a high affinity to the CB1-receptor (table 4 for 2 different experiments in rats).

**Table 4.**

| Compound according to example | $IC_{50}$ [nM] | $pIC_{50}$ |
|---|---|---|
| 1 (Compound A) | 16.1 | |
| 1 (Compound A) | 22 | 7.66 ± 0.08 |

The antagonism of compound A to the CB1-receptor was determined according to the method described in Pharmacological methods, part VI (table 5).

**Table 5**

| Compound according to example | Antagonism [%] |
|---|---|
| 1 (Compound A) | 106 |

**B) In-Vivo Data**

**Effect of continued administration of the compound A on body weight in rats**

The effect of continued administration of the compound A on body weight in rats was determined as described in the section Pharmacological methods, part V.

The number of animals was 10 for the (R)-N-piperidinyl-5-(4-chlorophenyl)-1-(2,4-dichlorophenyl)-4,5-dihydro-1H-pyrazole-3-carboxamide group (Compound A) and 9 for the racemic N-piperidinyl-5-(4-chlorophenyl)-1-(2,4-dichlorophenyl)-4,5-dihydro-1H-pyrazole-3-carboxamide group (Compound X) and for the vehicle group. In the vehicle group one animal died whereas in the racemic group one animal was excluded because its weight decreased during 5 days in the post-treatment period. All results are expressed as mean ± S.E.M.

The dependence of body-weight (expressed as a percentage relative to day 8) on time (number of days after inclusion in the study) and treatment was assessed by a two-way (time, treatment) analysis of variance (ANOVA) with repeated measures on the time factor. The analysis was performed on periods 2 and 3, separately, and considering the first or second week in each period. The differences between pairs of curves (treatments) were analyzed using appropriate contrasts within the ANOVA test. The possible interaction between time and treatment was also considered including the Greenhouse-Geisser (G-G) correction. A value of $P < 0.05$ was considered significant.

**Figure 1** shows the effect of continued administration (i.p. once daily) of racemic N-piperidinyl-5-(4-chlorophenyl)-l -(2,4-dichlorophenyl)-4,5-dihydro-1H-pyrazole-3-carboxamide **(Cpd. X),** (R)-N-piperidinyl-5-(4-chlorophenyl)-1-(2,4-dichlorophenyl)-4,5-dihydro-1H-pyrazole-3-carboxamide **(Cpd. A)** and vehicle **(Vehicle)** on body weight in rats. The (R)-compound according to example 1 shows a stronger action than the racemate as well as a slower recovery of weight.

**Further In-Vivo-Studies:**

In a series of further experiments the effects of compound A ((R)-N-piperidinyl-5-(4-chlorophenyl)-1-(2,4-dichlorophenyl)-4,5-dihydro-1H-pyrazole-3-carboxamide) on food intake, body weight, adiposity, dyslipidaemia and insulin resistance in rats that have become obese through the excessive consumption of highly palatable foods sources was tested.

**Animals**

The experiment was performed on female Wistar rats (originally in the weight range 250-300 g). The rats were housed in pairs in polypropylene cages with solid floors and sawdust bedding at a temperature of 21±4°C and 55±20% humidity. Animals were maintained on a reverse phase light-dark cycle (lights off for 8 h from 09.30-17.30 h) during which time the room was illuminated by red light. Animals had free access to powdered high fat diet (VRF1 plus 20% lard), ground chocolate, ground peanuts and tap water at all times. The three different diets were contained in separate glass feeding jars with aluminum lids with a 3-4 cm hole cut in it to allow access to the food. Animals were housed in pairs for twelve weeks for the induction of obesity. Animals were then housed singly in polypropylene cages with wire grid floors to enable the food intake of each rat to be recorded. Polypropylene trays with cage pads were placed beneath each cage to detect any food spillage. Animals were accustomed to these new conditions (individual housing and the wire-grid floor cages) for at least 14 days before the start of the baseline readings.

**Experimental procedures for the feeding study**

Approximately a week before the start of the study, animals were weighed (to the nearest 0.1 g using an electronic top-pan balance) and allocated into 3 weight-matched treatment groups, each containing 9-10 animals. Following a 7 day baseline run-in period, during which the animals were dosed by the appropriate route (eg orally) once a day with vehicle (0.5% hydroxy-propyl-methylcellulose [HPMC]; 1 ml/kg) or the test drug, ie Compound (Cpd.) A (10 and 30 mg/kg po) once daily for 28 days.

Food intake and body weight were measured once daily. Variations in body weight and energy levels of the different types of food were accounted for by expressing the food intake results in terms of kJ/kg rat weight.

Statistical comparisons between the body weights and daily food intakes of the different treatment groups have been made by analysis of covariance followed by Dunnett's test to compare each treatment group with the vehicle-treated controls. For the body weight data (body weights each day), the weights of the animals on Day 1 (ie immediately before the first drug treatment) were used as the covariate. Thus, means were adjusted for differences in body weight between the groups on Day 1 and standard errors of the mean (SEM) were calculated from the residuals of the statistical model. All mean body weight values on Day 1 are identical (and SEM are therefore zero) because the analysis of covariance adjusts them all to equal the 'grand mean' of the values of this day.

**Determination of carcass fat content**

Total ether-extractable fat in the freeze-dried samples was estimated by the Soxhlet principle using a Foss Soxtec HT2 system. The protocol employed was based on the manufacturer's recommendations for meat samples, although freeze-dried samples obviated the necessity for either a fat hydrolysis step or mixing the sample with sand to ensure efficient extraction.

Samples of approximately 1 g were accurately weighed into 26 mm x 60 mm cellulose extraction thimbles which were then plugged with approximately 0.8 g cotton wool. Sample fat was extracted into pre-weighed (including a few boiling chips) standard metal extraction cups using 70 ml of petroleum ether at 40-60°C, a circulator temperature of 115°C and a cooling flow rate of 2 l/min. A boiling stage of 25 min was employed, followed by a 40 min wash stage. A 10 min drying stage sufficed to remove ether from the extraction cups.

Percentage fat in the freeze-dried sample was calculated as follows:

$$\%\ \text{fat} = [100 \times (\text{final cup weight} - \text{initial cup weight})]/\text{sample weight}$$

Percentage fat in the original sample was calculated using the % water as follows:

$$\% \text{ fat in original sample} = \% \text{ fat} \times [(100 - \% \text{ water})/100]$$

**Plasma analyses**

Terminal blood samples via cardiac puncture were taken from the animals immediately after death into Sarstedt 4.5 ml lithium-heparin tubes (Cat No. 32.331). Tubes were then centrifuged at 1,500 g for 5 minutes at 4°C and the plasma removed and immediately frozen on dry ice (as 3 aliquots per sample in sealed Eppendorf tubes). Plasma samples were stored at -75°C until required for analysis. All determinations were performed on plasma samples that had been thawed only once.

**Leptin assay**

Plasma leptin concentration was determined using Assay Designs rat leptin enzyme immunometric assay kits (Cat No. 900-015) according to the manufacturer's regular protocol. Unknown samples were diluted 100 times using leptin assay buffer prior to assay in duplicate. Optical density at 450 nm was determined using a Molecular Devices VERSAmax tunable microplate reader and readings transferred to GraphPad Prism. A second order polynomial curve was fitted to the calibration data and unknown values extrapolated from this curve fit.

**Insulin assay**

Plasma insulin concentration was determined using Mercodia ultrasensitive rat insulin ELISA kits according to the manufacturers 50 μl sample protocol. Unknown samples were diluted 20 times using the zero standard prior to assay in duplicate: Optical density at 450 nm was determined using a Molecular Devices VERSAmax tunable microplate reader and readings transferred to GraphPad Prism. A cubic spline curve was fitted to the calibration data and unknown values extrapolated from this curve fit.

**Results**

Chronic effects of Compound A ((R)-N-piperidinyl-5-(4-chlorophenyl)-(2,4-dichlorophenyl)-4.5-dihydro-1H-pyrazole-3-carboxamide) (10 and 30 mg/kg po) on the consumption of individual macronutrient sources

In rats that had become obese after excessive consumption of a palatable "cafeteria" diet consisting of high-fat chow, ground dairy milk chocolate and ground roasted salted peanuts, repeated oral administration of Compound A in comparison with the vehicle-treated control group resulted in that palatable food intake was significantly reduced by both the 10 and 30 mg/kg doses on the, first day of drug administration and daily food intake remained significantly lower than the control group on Days 2 to 7 with the lower dose and on Days 2 to 10 and Day 15 for the higher dose (Figure 2).

Results in Figure 2 are means (adjusted for differences between the food intakes of the different treatment groups at baseline (average of Days -6 to 0)) ± SEM (calculated from the residuals of the statistical model), n=9-10. Multiple comparisons against the vehicle-treated control group were by Dunnett's test. Significant differences from the control group are denoted by *p<0.05 (p levels have only been given at one level for clarity).

Chronic effects of Compound A ((R)-N-piperidinyl-5-(4-chlorophenyl)-1-(2,4-dichlorophenyl)-4,5-dihydro-1H-pyrazole-3-carboxamide) (10 and 30 mg/kg po) on the body weights of obese rats given access to highly palatable, macronutrient food sources

The body weights of the vehicle-treated control group slowly increased throughout the study (Figure 3). Chronic administration of Compound A (10 and 30 mg/kg po) produced dose-related decreases in body weight (Figure 3). The body weights of animals given either dose of Compound A were significantly lower than the control group on Day 2 (ie 24 h after the first dose of the drug) and on every other day of the drug treatment period (Figure 3). Thus, at the end of the 28 day drug treatment period, the body weights of dietary-induced obese, female Wistar rats given Compound A (10 and 30 mg/kg po) were 12.0% and 19.0% lower, respectively, than those of the vehicle-treated controls (Figure 3).

Results shown in Figure 3 are means (adjusted for differences between the body weights of the different

treatment groups at baseline (Day 1)) ± SEM (calculated from the residuals of the statistical model), n=9-10. Numbers represent % reduction compared to the control group on Day 29 (ie after 28 days of treatment). Multiple comparisons against the vehicle-treated control group were by Dunnett's test. Significant differences from the control group are denoted by *p<0.05 (p levels have only been given at one level for clarity).

Chronic effects of Compound A ((R)-N-piperidinyl-5-(4-chlorophenyl) -(2.4-dichlorophenyl)-4,5-dihydro-1H-pyrazole-3-carboxamide) (10 and 30 mg/kg po) on the adiposity of obese rats given access to highly palatable, macronutrient food sources

The effects of chronic oral administration of Compound A for 28 days on body composition in regards to fat are shown in Figures 4 (expressed as g per rat).

Repeated administration with Compound A (10 and 30 mg/kg po) produced a large decrease in body fat of 42.8 g and 67.8 g, respectively, compared to the vehicle-treated control group (Figure 4). Results in Figure 4 are expressed as treatment group means (n=9-10 and adjusted for differences between the groups in body weight at baseline) and SEM (calculated from the residuals of the statistical model). Statistical comparisons were by ANCOVA (baseline body weight as covariate) followed by Dunnett's test. Significant differences are denoted by ***p<0.001, **p<0.01, *p<0.05.

Consistent with the relative reductions in body fat shown in Figure 4, plasma leptin levels were decreased by 36.1%, 61.7% after Compound A (10 and 30 mg/kg po) when compared to the vehicle-treated control group (Figure 5). Results in Figure 5 are expressed as treatment group means and SEM (all n=9-10). Analysis of covariance was used using baseline body weight and bleeding order (animal number) as covariate. The Rank transformation was used. Multiple comparisons against vehicle were by Dunnetts' test. Significant differences are denoted by *p.05 and ***p<0.001

Chronic effects of Compound A ((R)-N-piperidinyl-5-(4-chlorophenyl)-1-(2,4-dichlorophenyl )-4,5-dihydro-1H-pyrazole-3-carboxamide) (10 and 30 mg/kg po) on plasma insulin concentrations in obese rats given access to highly palatable, macronutrient food sources

The effects of Compound A (10 and 30 mg/kg po) on plasma insulin levels are shown in Figure 6.

Compound A (10 and 30 mg/kg po) significantly decreased plasma insulin levels by 38.9% and 41.8%, respectively compared to the vehicle-treated control group (Figure 6). Results in Figure 6 are expressed as treatment group means and SEM (all n=9-10). Analysis of covariance was used using baseline body weight and bleeding order (animal number) as covariate. The Rank transformation was used. Multiple comparisons against vehicle were by Dunnetts' test. Significant differences are denoted by **p<0.01, ***p<0.001.

## Claims

1. Amorphous phase of (R)-N-piperidinyl-5-(4-chlorophenyl)-1-(2,4-dichlorophenyl)-4,5-dihydro-1H-pyrazole-3-carboxamide.

2. Amorphous phase of (R)-N-piperidinyl-5-(4-chlorophenyl)-1-(2,4-dichlorophenyl)-4,5-dihydro-1H-pyrazole-3-carboxamide according to claim 1, **characterized in that** it shows a glass transition in the DSC analysis at 64.5 ± 4 °C, preferably at 64.5 ± 3 °C, more preferably at 64.5 ± 2 °C or also between 63 and 66° C.

3. Amorphous phase of (R)-N-piperidinyl-5-(4-chlorophenyl)-1-(2,4-dichlorophenyl)-4,5-dihydro-1H-pyrazole-3-carboxamide according to claim 1, **characterized in that** it decomposes at or above 300 °C.

4. Amorphous phase of (R)-N-piperidinyl-5-(4-chlorophenyl)-1-(2,4-dichlorophenyl)-4,5-dihydro-1H-pyrazole-3-carboxamide according to claim 1, **characterized in that** it shows in the FTIR spectrum peaks, preferably intense and/or broad peaks at 1656 ± 5 $cm^{-1}$ and/or 1473 ± 5 $cm^{-1}$ and no peaks, preferably no intense peaks, at 3325 ± 10 $cm^{-1}$.

5. Amorphous phase of (R)-N-piperidinyl-5-(4-chlorophenyl)-1-(2,4-dichlorophenyl)-4,5-dihydro-1H-pyrazole-3-carboxamide according to claim 1, **characterized in that** it shows in the FTRaman spectrum bands, preferably intense and/or broad bands, at 1666 ± 5 $cm^{-1}$.

6. Process for the manufacture of the amorphous phase of (R)-N-piperidinyl-5-(4-chlorophenyl)-1-(2,4-dichlorophenyl)-4,5-dihydro-1H-pyrazole-3-carboxamide according to any of claims 1 to 5, wherein (R)-N-piperidinyl-5-(4-chlorophenyl)-1-(2,4dichlorophenyl)-4,5-dihydro-1H-pyrazole-3-carboxamide is melted, preferably at a temperature between 78 and 88°C, and subsequently cooled below the melting point, preferably to room temperature.

**7.** Medicament comprising at least the amorphous phase of (R)-N-piperidinyl-5-(4-chlorophenyl)-1-(2,4-dichlorophenyl)-4,5-dihydro-1H-pyrazole-3-carboxamide according to any of claims 1 to 5 and optionally one or more pharmaceutically acceptable excipients.

**8.** Use of the amorphous phase of (R)-N-piperidinyl-5-(4-chlorophenyl)-1-(2,4-dichlorophenyl)-4,5-dihydro-1H-pyrazole-3-carboxamide according to any of claim 1 to 5 for the preparation of a medicament for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

**9.** Use of the amorphous phase of (R)-N-piperidinyl-5-(4-chlorophenyl)-l-(2,4-dichlorophenyl)-4,5-dihydro-1H-pyrazole-3-carboxamide according to any of claim 1 to 5 for the preparation of a medicament for the prophylaxis and/or treatment of food intake disorders, preferably bulimia, anorexia, cachexia, obesity, type II diabetus mellitus (non-insuline dependent diabetes mellitus), more preferably obesity.

**10.** Use of the amorphous phase of (R)-N-piperidinyl-5-(4-chlorophenyl)-1-(2,4-dichlorophenyl)-4,5-dihydro-1H-pyrazole-3-carboxamide according to any of claims 1 to 5, for the preparation of a medicament for the prophylaxis and/or treatment of psychosis.

**11.** Use of the amorphous phase of (R)-N-piperidinyl-5-(4-chlorophenyl)-1-(2,4-dichlorophenyl)-4,5-dihydro-1H-pyrazole-3-carboxamide according to any of claims 1 to 5 for the preparation of a medicament for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

**12.** Use of the amorphous phase of (R)-N-piperidinyl-5-(4-chlorophenyl)-1-(2,4-dichlorophenyl)-4,5-dihydro-1H-pyrazole-3-carboxamide according to any of claims 1 to 5, for the preparation of a medicament for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

**13.** Use of the amorphous phase of (R)-N-piperidinyl-5-(4-chlorophenyl)-1-(2,4-dichlorophenyl)-4,5-dihydro-1H-pyrazole-3-carboxamide according to any of claims 1 to 5, for the preparation of a medicament for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

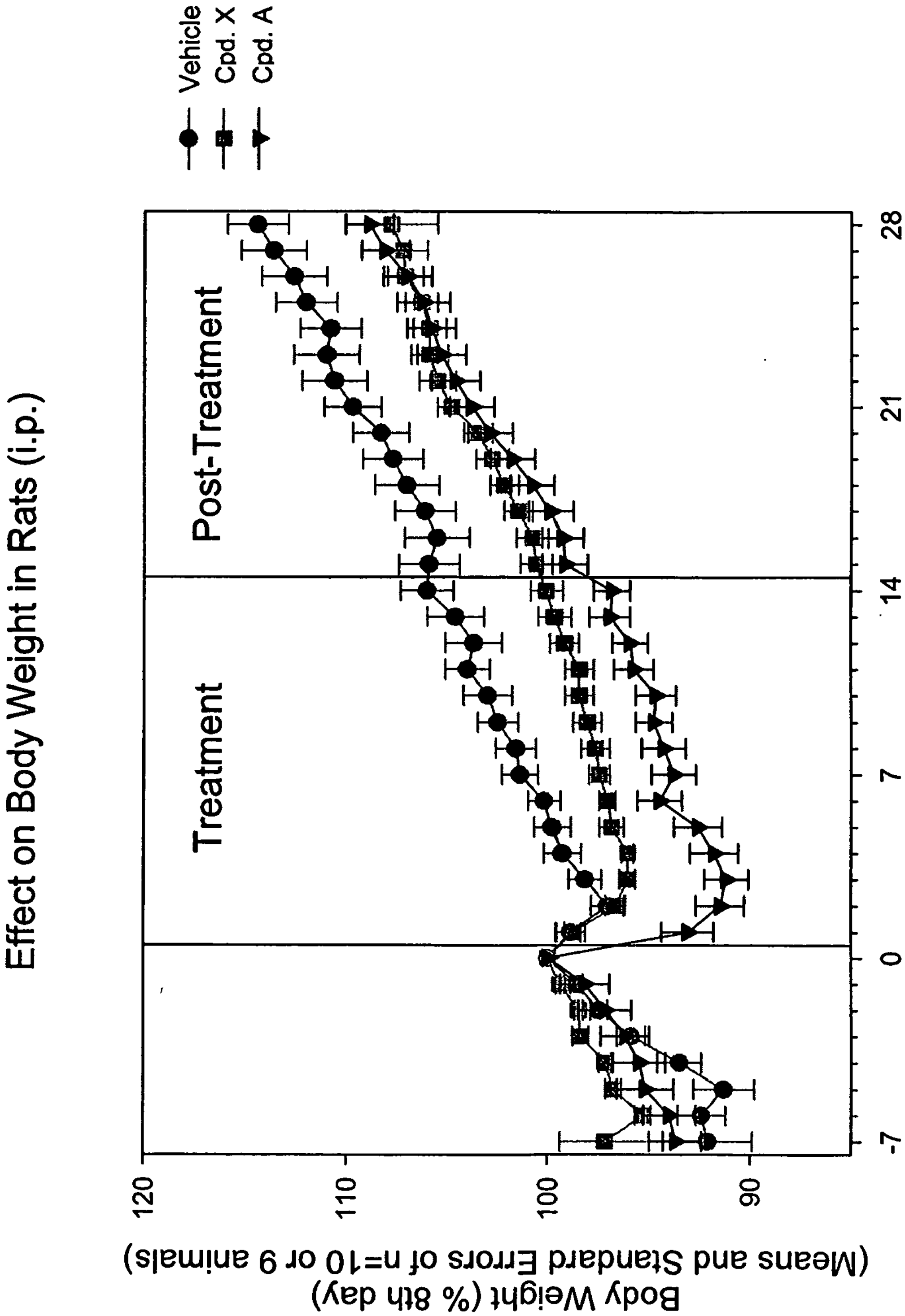
Effect on Body Weight in Rats (i.p.)
Treatment
Post-Treatment
Vehicle
Cpd. X
Cpd. A
Body Weight (% 8th day)
(Means and Standard Errors of n=10 or 9 animals)
120
110
100
90
-7
0
7
14
21
28
Time (days)

Figure 1

Figure 2

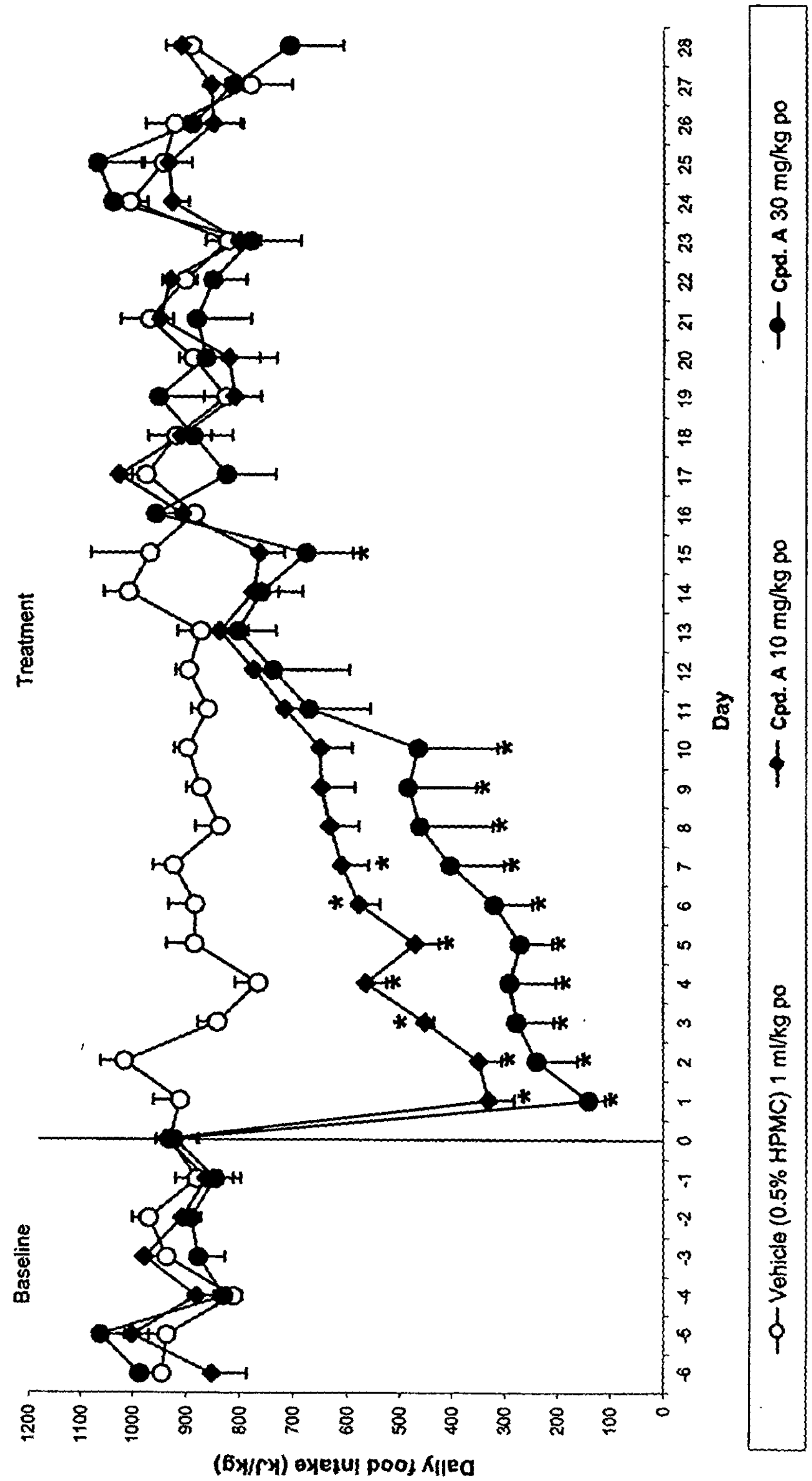
Baseline
Treatment
Daily food intake (kJ/kg)
Day
Vehicle (0.5% HPMC) 1 ml/kg po
Cpd. A 10 mg/kg po
Cpd. A 30 mg/kg po

Figure 3

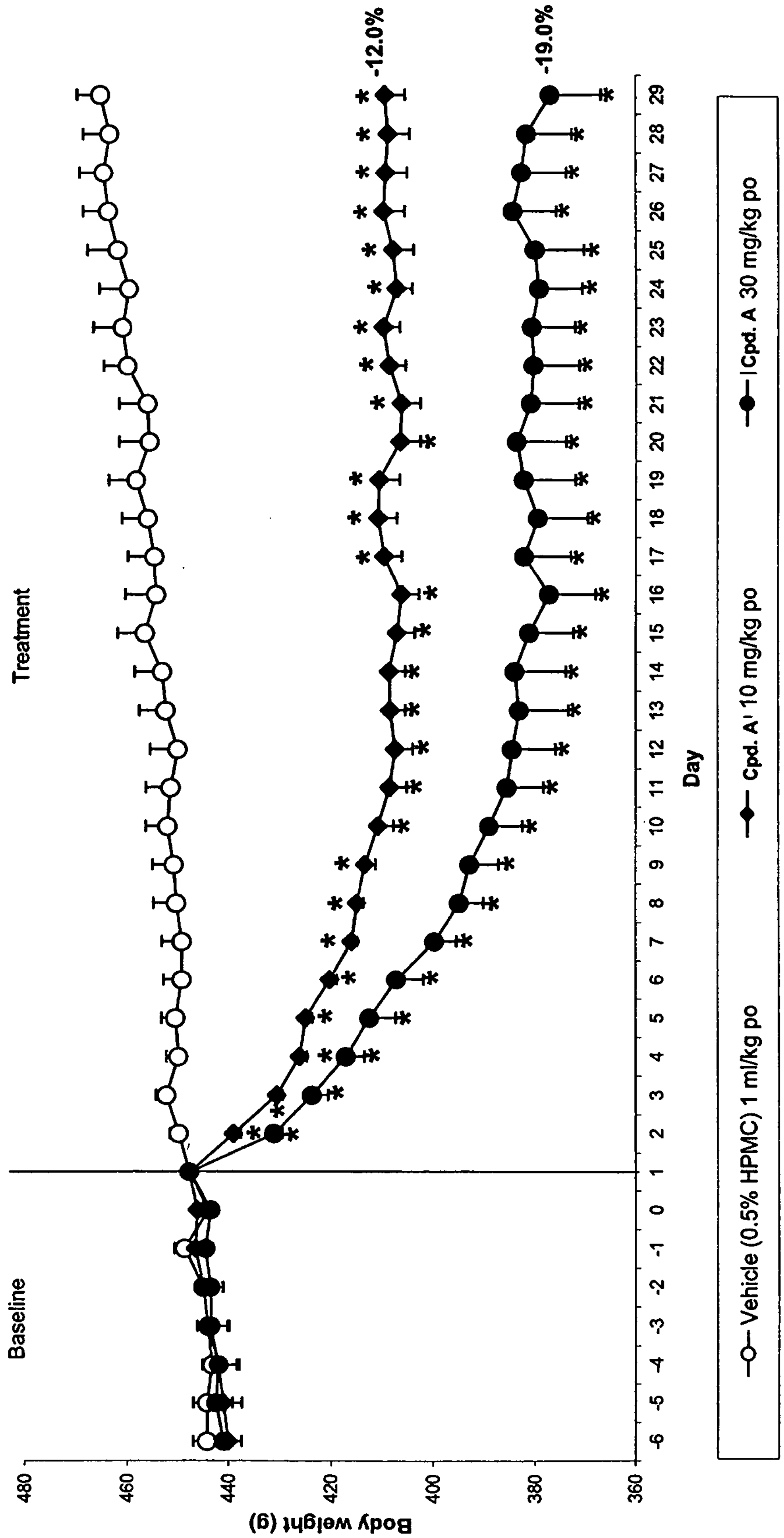
Baseline
Treatment
-12.0%
-19.0%
Body weight (g)
480
460
440
420
400
380
360
Day
-6
-5
-4
-3
-2
-1
0
1
2
3
4
5
6
7
8
9
10
11
12
13
14
15
16
17
18
19
20
21
22
23
24
25
26
27
28
29
Vehicle (0.5% HPMC) 1 ml/kg po
Cpd. A' 10 mg/kg po
Cpd. A 30 mg/kg po

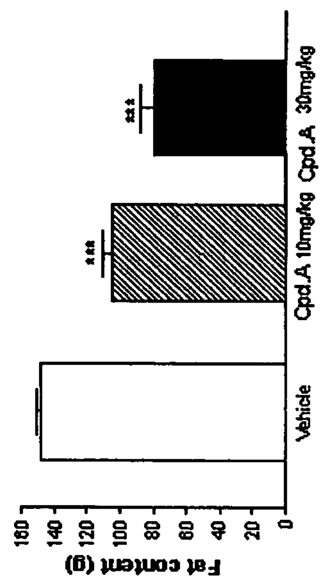
Fat content (g)
0
20
40
60
80
100
120
140
160
180
Vehicle
Cpd.A 10mg/kg
Cpd.A 30mg/kg
***
***

Figure 4

Figure 5:

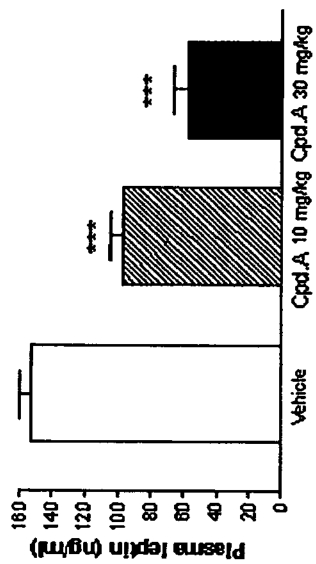
Plasma leptin (ng/ml)
0
20
40
60
80
100
120
140
160
Vehicle
Cpd.A 10 mg/kg
Cpd.A 30 mg/kg
***
***

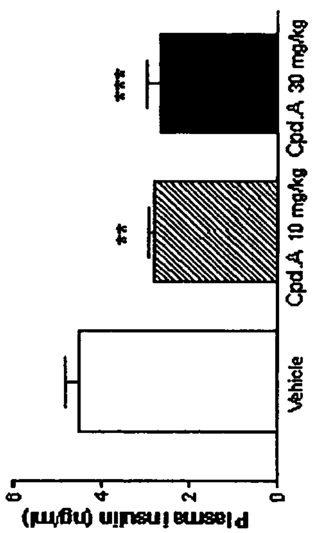


Figure 6:

Figure 7:

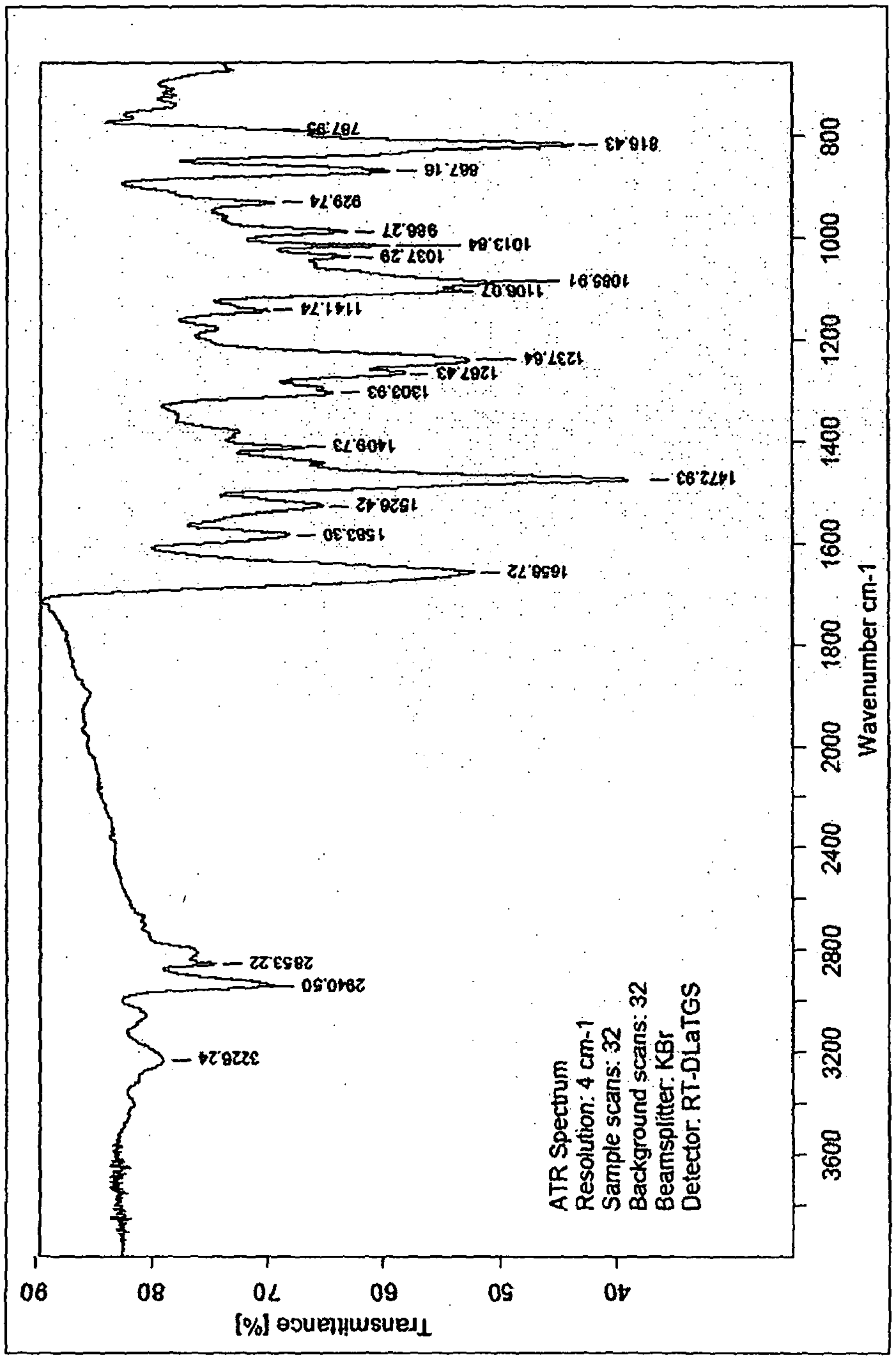
Transmittance [%]
90
80
70
60
50
40
Wavenumber cm-1
3600
3200
2800
2400
2000
1800
1600
1400
1200
1000
800
ATR Spectrum
Resolution: 4 cm-1
Sample scans: 32
Background scans: 32
Beamsplitter: KBr
Detector: RT-DLaTGS
3226.24
2940.50
2853.22
1656.72
1563.30
1526.42
1472.93
1409.73
1303.93
1267.43
1237.64
1141.74
1106.07
1085.91
1037.29
1013.84
986.27
929.74
867.16
816.43
787.95

Figure 8:

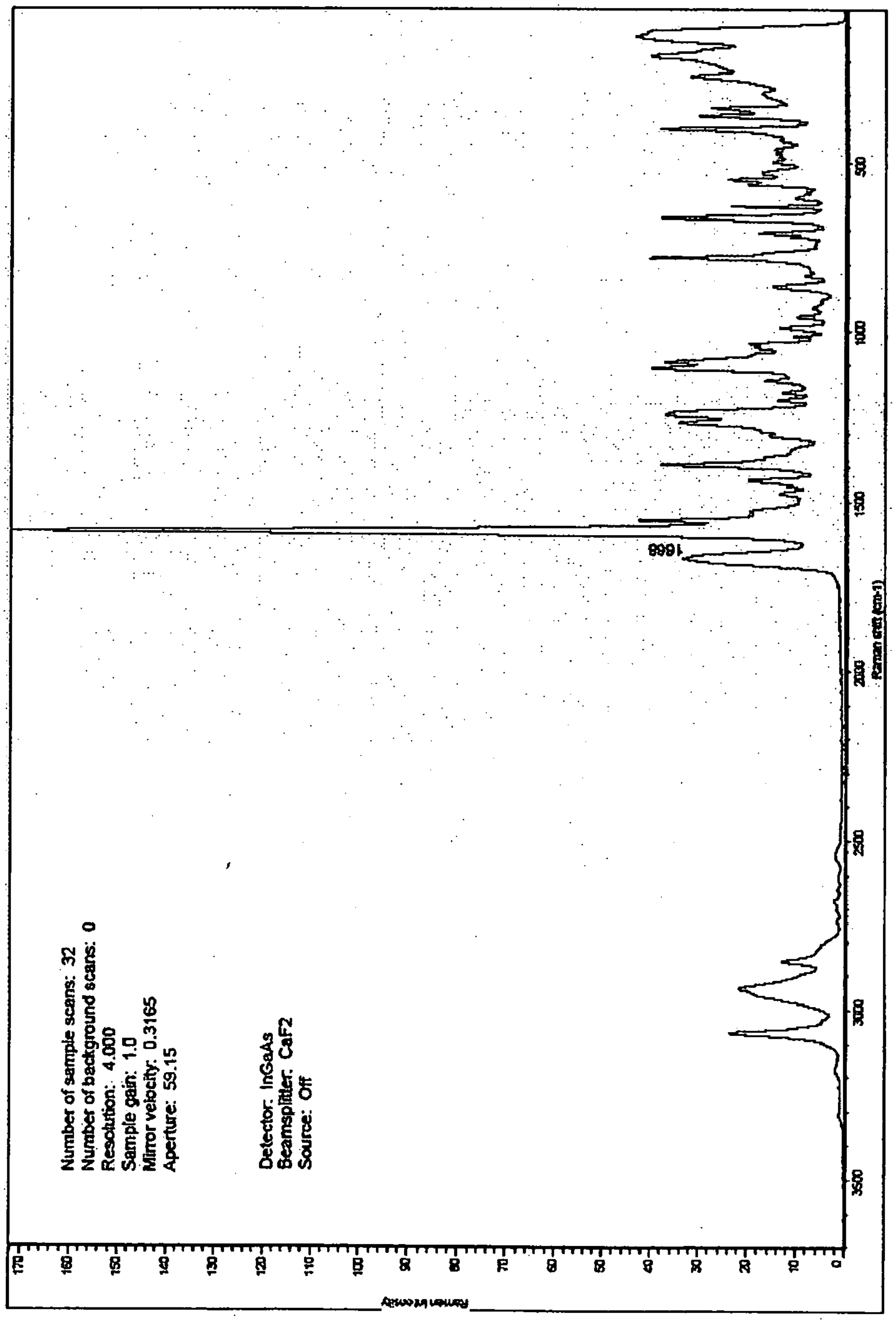
Number of sample scans: 32
Number of background scans: 0
Resolution: 4.000
Sample gain: 1.0
Mirror velocity: 0.3165
Aperture: 59.15
Detector: InGaAs
Beamsplitter: CaF2
Source: Off
1668
Raman intensity
Raman shift (cm-1)

European Patent Office

# EUROPEAN SEARCH REPORT

Application Number
EP 07 38 4006

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2005/077909 A (ESTEVE LABOR DR [ES]; CUBERES ALTISEN ROSA [ES]; GUITIERREZ SILVA BONI) 25 August 2005 (2005-08-25) * claims; example 1 * | 1-13 | INV. C07D231/06 A61K31/4155 A61P25/00 |
| A | MESCHLER J P ET AL: "Inverse agonist properties of N-(piperidin-1-yl)-5-(4-chlorophenyl)-1-(2, dichlorophenyl)-4-methyl-1H-pyrazole-3-carboxamide HCl (SR141716A) and 1-(2-chlorophenyl)-4-cyano-5-(4-methoxyphenyl)-1H-pyr azole-3-carboxylic acid phenylamide (CP-272871) for the CB1 cannabinoid receptor" BIOCHEMICAL PHARMACOLOGY, PERGAMON, OXFORD, GB, vol. 60, 2000, pages 1315-1323, XP002262541 ISSN: 0006-2952 * the whole document * | 1-13 | |
| E | EP 1 757 589 A (ESTEVE LABOR DR [ES]) 28 February 2007 (2007-02-28) * the whole document * | 1-13 | TECHNICAL FIELDS SEARCHED (IPC) C07D A61K A61P |
| E | EP 1 743 888 A (ESTEVE LABOR DR [ES]) 17 January 2007 (2007-01-17) * examples * | 1-13 | |

The present search report has been drawn up for all claims

1

| Place of search | Date of completion of the search | Examiner |
|---|---|---|
| The Hague | 5 June 2007 | Bosma, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT ON EUROPEAN PATENT APPLICATION NO.** EP 07 38 4006

This annex lists the patent family members relating to the patent documents cited in the above-mentioned European search report. The members are as contained in the European Patent Office EDP file on
The European Patent Office is in no way liable for these particulars which are merely given for the purpose of information.

05-06-2007

| Patent document cited in search report | Publication date | Patent family member(s) | Publication date |
|---|---|---|---|
| WO 2005077909 A | 25-08-2005 | AU 2005212817 A1 | 25-08-2005 |
| | | CA 2556568 A1 | 25-08-2005 |
| | | KR 20060135815 A | 29-12-2006 |
| EP 1757589 A | 28-02-2007 | NONE | |
| EP 1743888 A | 17-01-2007 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005077909 A
- WO 2005077911 A


### Non-patent literature cited in the description


- **HOLLISTER.** *Pharm. Rev.,* 1986, vol. 38, 1-20
- **RENY ; SINGHA.** *Prog. Drug. Res,* 1991, vol. 36, 71-114
- **CONSROE ; SANDYK.** Marijuana/Cannabinoids, Neurobiology and Neurophysiology. CRC Press, 1992, vol. 459
- **ECKEL et al.** *The Lancet,* 2005, vol. 365, 1415-1428
- **ALBERTI et al.** *Diabet. Med,* 1998, vol. 15, 539-53
- *JAMA,* 2001, vol. 285, 2486-97
- **PAGOTTO ; PASQUALI.** *The Lancet,* 2005, vol. 365, 1363, 1364
- **RUTH A. ROSS ; HEATHER C. BROCKIE et al.** Agonist-inverse agonist characterization at CB1 and CB2 cannabinoid receptors of L-759633, L759656 and AM630. *British Journal of Pharmacology,* 1999, vol. 126, 665-672
- **GOVAERTS et al.** *Eur J Pharmac Sci,* 2004, vol. 23, 233-243
- **A. C. HOWLETT et al.** International Union of Pharmacology XXVII. Classification of Cannabinoid Receptors. *Pharmacol Rev,* 2002, vol. 54, 161-202
- **DAVID R. COMPTON et al.** In-vivo Characterization of a Specific Cannabinoid Receptor Antagonist (SR141716A) :Inhibition of Tetrahydrocannbinol- induced Responses and Apparent Agonist Activity. *J. Pharmacol. Exp. Ther,* 1996, vol. 277 (2), 586-594
- **WOOLFE D. et al.** The evaluation of analgesic action of pethidine hydrochloride (Demerol. *J. Pharmacol. Exp. Ther,* 1944, vol. 80, 300-307
- **DESMET L. K. C. et al.** Anticonvulsive properties of Cinarizine and Flunarizine in Rats and Mice. *Arzneim. - Forsch. (Frug Res,* 1975, vol. 25, 9
- **DAVID R. COMPTON et al.** In-vivo Characterization of a Specific Cannabinoid Receptor Antagonist (SR141716A) Inhibition of Tetrahydrocannbinol- induced Responses and Apparent Agonist Activity. *J. Pharmacol Exp Ther,* 1996, vol. 277 (2), 586-594
- **ALPERMANN H. G. et al.** Pharmacological effects of Hoe 249: A new potential antidepressant. *Drugs Dev. Res,* 1992, vol. 25, 267-282
- **G. COLOMBO et al.** Appetite Suppression and Weight Loss after the Cannabinoid Antagonist SR 141716. *Life Sciences,* 1998, vol. 63 (8), 113-117
- **E.T. TZAVARA et al.** The CB1 receptor antagonist SR141716A selectively increases monoaminergic neurotransmission in the medial prefrontal cortex: implications for therapeutic actions. *Br. J. Pharmacol,* 2003, vol. 138 (4), 544-53
- **LUMENG, L et al.** Different sensitivities to ethanol in alcohol-preferring and-nonpreferring rats. *Pharmacol, Biochem Behav,* 1982, vol. 16, 125-130
- **MIDDAUGH et al.** Ethanol Consumption by C57BU6 Mice : Influence of Gender and Procedural Variables. *Alcohol,* 1999, vol. 17 (3), 175-183
- **LE et al.** Alcohol Consumption by C57BU6, BALA/c, and DBA/2 Mice in a Limited AccessParadigm. *PharmacologyBiochemistry and Behavior,* 1994, vol. 47, 375-378
- **VASTOLA et al.** *Physiol. Behav,* 2002, vol. 77, 107-114
- **BROWER et al.** *Brain Res,* 2002, vol. 930, 12-20
- **PORSOLT et al.** *Nature,* 1977, vol. 266, 730-732
- **STEM et al.** *Psychopharmacology,* 1985, vol. 85, 367-370
- **SWERDLOW ; GEYER.** *Schizophrenia Bulletin,* 1998, vol. 24, 285-301
- **PELLOW ; FILE.** *Pharm. Biochem. Behav,* 1986, vol. 24, 525-529
- **ANDERSSON et al.** *J. Urol,* 1999, vol. 161, 1707-17
- **HALE et al.** *Int. J. Impot. Res,* 2003, vol. 15 (5), 75-79